(12) # EUROPEAN PATENT APPLICATION

(21) Application number: 88307889.1

(22) Date of filing: 25.08.88

(51) Int. Cl.⁴: C07K 13/00 , C07K 7/10 , C07K 7/08 , C12P 21/02 , C12N 15/00 , G01N 33/569 , A61K 37/02 , //A61K39/21

(30) Priority: 27.08.87 US 90080

(43) Date of publication of application: 08.03.89 Bulletin 89/10

(84) Designated Contracting States:
AT BE CH DE ES FR GB GR IT LI LU NL SE

(71) Applicant: REPLIGEN CORPORATION
101 Binney Street
Cambridge Massachusetts 02142(US)

(72) Inventor: Rusche, James R.
18 Brigham Road
Framingham MA 01701(US)
Inventor: Putney, Scott D
5 Epping Street
Arlington, MA 02174(US)
Inventor: Javaherian, Kashayar
27 Webster Road
Lexington, MA 02173(US)
Inventor: Farley, John
261 Culver Road No. 9
Rochester, NY 14607(US)
Inventor: Grimaila, Raymond
311 Washington Street
Somerville, MA 02174(US)
Inventor: Lynn, Debra
11 Allen Street, Apt. 11
Arlington, MA 02138(US)
Inventor: Petro, Joan
59 Sacramento Street
Cambridge, MA 02138(US)
Inventor: O'Keeffe, Thomas
85 Mapleton Street
Brighton, MA 02135(US)

(74) Representative: Perry, Robert Edward et al
GILL JENNINGS & EVERY 53-64 Chancery
Lane
London WC2A 1HN(GB)

(54) Novel HIV proteins and peptides useful in the diagnosis, prophylaxis or therapy of AIDS.

(57) Novel HIV proteins and peptides can be used in the diagnosis, prophylaxis or therapy of AIDS. They are considered to be especially useful to prepare vaccines for HIV.

# NOVEL HIV PROTEINS AND PEPTIDES USEFUL IN THE DIAGNOSIS, PROPHYLAXIS OR THERAPY OF AIDS

## Background of the Invention

Human immunodeficiency virus (HIV), human T-cell lymphotropic virus III (HTLV-III), lymphadenopathy-associated virus (LAV), or AIDS-associated retrovirus (ARV) has been identified as the cause of acquired immune deficiency syndrome (AIDS) (Popovic, M., Sarngadharan, M.G., Read, E. and Gallo, R.C. [1984] Science 224:497-500). The virus displays tropism for the OKT4$^+$ lymphocyte subset (Klatzmann, D., Barre-Sinoussi, F., Nugeyre, M.T., Dauguet, C., Vilmer, E., Griscelli, C., Brun-Vezinet, F., Rouzioux, C., Gluckman, J.D., Chermann, J.C. and Montagnier, L. [1984] Science 225:59-63). Antibodies against HIV proteins in the sera of most AIDS and AIDS related complex (ARC) patients, and in asymptomatic people infected with the virus (Sarngadharan, M.G., Popovic, M., Bruch, L., Schupbach, J. and Gallo, R.C. [1984] Science 224:506-508) have made possible the development of immunologically based tests that detect antibodies to these antigens. These tests are used to limit the spread of HIV through blood transfusion by identifying blood samples of people infected with the virus. Diagnostic tests currently available commercially use the proteins of inactivated virus an antigens.

In addition to allowing new approaches for diagnosis, recombinant DNA holds great promise for the development of vaccines against both bacteria and viruses (Wilson, T. [1984] Bio/Technology 2:29-39). The most widely employed organisms to express recombinant vaccines have been E. coli, S. cerevisiae and cultured mammalian cells. For example, subunit vaccines against foot and mouth disease (Kleid, D.G., Yansura, D., Small, B., Dowbenko, D., Moore, D.M., Brubman, M.J., McKercher, P.D., Morgan, D.O., Robertson, B.H. and Bachrach, H.L. [1981] Science 214:1125-1129) and malaria (Young, J.F., Hockmeyer, W.T., Gross, M., Ripley Ballou, W., Wirtz, R.A., Trosper, J.H., Beaudoin, R.L., Hollingdale, M.R., Miller, L.M., Diggs, C.L. and Rosenberg, M. [1985] Science 228:958-962) have been synthesized in E. coli. Other examples are hepatitis B surface antigen produced in yeast (McAleer, W.J., Buynak, E.B., Maigetter, R.Z., Wampler, D.E., Miller, W.J. and Hilleman, M.R. [1984] Nature 307: 178-180) and a herpes vaccine produced in mammalian cells (Berman, P.W., Gregory, T., Chase, D. and Lasky, L.A. [1984] Science 227:1490-1492).

The entire HIV envelope or portions thereof have been used to immunize animals. Both the native gp120 (Robey et al. [1986] Proc. Natl. Acad. Sci. 83:7023-7027; Matthews et al., [1986] Proc. Natl. Acad. Sci 83:9709-9713) and recombinant proteins (Laskey, et al., [1986] Science 233:209-212; Putney et al., [1986] Science 234:1392-1395) elicit antibodies that can neutralize HIV in cell culture. However, all of these immunogens elicit antibodies that neutralize only the viral variant from which the subunit was derived. Therefore, a novel vaccine capable of protecting against multiple viral variants would contain subunits from multiple variants.

Diagnostic kits or therapeutic agents using viral proteins isolated from virus infected cells or recombinant proteins would contain epitopes specific to the viral variant from which they were isolated. Reagents containing proteins from multiple variants would have the utility of being more broadly reactive due to containing a greater diversity of epitopes. This would be advantageous in the screening of serum from patients or therapeutic treatment of patients.

Synthetic peptides can be advantageous as the active ingredient in a vaccine, therapeutic agent or diagnostic reagent due to the ease of manufacture and ability to modify their structure and mode of presentation.

Synthetic peptides have been used successfully in vaccination against foot and mouth disease virus (Bittle, et al. [1982] Nature 298:30-33); poliovirus (Emini et al. [1983] Nature 305:699); hepatitis B (Gerin et al. [1983] Proc. Natl. Acad. Sci. 80:2365-2369); and influenza (Shapira et al. [1984] Proc. Natl. Acad. Sci. 81:2461-2465).

There is a real need at this time to develop a vaccine for AIDS. Such a vaccine, advantageously, would be effective to immunize a host against the variant AIDS viruses.

## Brief Summary of the Invention

The subject invention concerns novel HIV proteins and peptides which can be used in the diagnosis, prophylaxis or therapy of AIDS. Further, the HIV envelope proteins and peptides of the invention can be used to stimulate a lymphocyte proliferative response in HIV-infected humans. This then would stimulate the immune system to respond to HIV in such individuals and, therefore, the envelope proteins and peptides can provide protection and be of therapeutic value. The proteins and peptides of the invention are identified herein by both their amino acid sequences and the DNA encoding them. Thus, they can be prepared by known chemical synthetic procedures, or by recombinant DNA means. Table 1 is a schematic of the synthetic peptides of the invention. These peptides are referenced to the N-terminal region of an HIV protein denoted $PB1_{IIIB}$.

More specifically, the novel proteins and peptides are as follows:

1. HIV 10 Kd fusion protein denoted Sub 1. The amino acid sequence of the HIV portion of Sub 1 is shown in Table 2 and the DNA sequence of the HIV portion of Sub 1 in Table 2A. The amino acid sequence of Sub 1 is shown in Table 2B and the DNA sequence in Table 2C.

2. HIV 18 Kd fusion protein denoted Sub 2. The amino acid sequence of the HIV portion of Sub 2 is shown in Table 3 and the DNA sequence of the HIV portion of Sub 2 in Table 3A. The entire amino acid sequence of Sub 2 is shown in Table 3B and the entire DNA sequence in Table 3C.

3. HIV 27 Kd fusion protein denoted $PB1_{RF}$. The amino acid sequence of the HIV portion of $PB1_{RF}$ is listed in Table 4 and the DNA sequence of the HIV portion of $PB1_{RF}$ is listed in Table 4A. The entire amino acid sequence and DNA sequence of $PB1_{RF}$ are in Tables 4B and 4C, respectively.

4. HIV 28 Kd fusion protein denoted $PB1_{MN}$. The amino acid sequence of the HIV portion of $PB1_{MN}$ is shown in Table 5 and the DNA sequence of the HIV portion of $PB1_{MN}$ is shown in Table 5A. The entire amino acid sequence and DNA sequence of $PB1_{MN}$ are shown in Tables 5B and 5C, respectively.

5. HIV 26 Kd fusion protein denoted $PB1_{SC}$. The amino acid sequence of the HIV portion of $PB1_{SC}$ is listed in Table 6 and the DNA sequence of the HIV portion of $PB1_{SC}$ is shown in Table 6A. The entire amino acid sequence and DNA sequence of $PB1_{SC}$ are shown in Tables 6B and 6C, respectively.

6. HIV 26 Kd fusion protein denoted $PB1_{WMJ2}$. The amino acid sequence of the HIV portion of $PB1_{WMJ2}$ is listed in Table 7 and the DNA sequence in Table 7A. The entire amino acid sequence and DNA sequence of $PB1_{WMJ2}$ are shown in Tables 7B and 7C, respectively.

7. Protein CNBr1. This protein contains the HIV env gene amino acids listed in Table 8 and the entire CNBr1 sequence in Table 8A.

8. Peptide No. 131 containing the HIV env gene amino acids listed in Table 9.

9. Peptide No. 132 containing the HIV env gene amino acids listed in Table 10.

10. Peptide No. 134 containing the HIV env gene amino acids listed in Table 11.

11. Peptide No. 135 containing the HIV env gene amino acids listed in Table 12.

12. Peptide No. 136 containing the HIV env gene amino acids listed in Table 13.

13. Peptide No. 138 containing the HIV env gene amino acids listed in Table 14.

Detailed Disclosure of the Invention

The proteins and peptides of the subject invention can be made by well-known synthesis procedures. Alternatively, these entities can be made by use of recombinant DNA procedures. Such recombinant DNA procedures are disclosed herein since they were, in fact, the procedures initially utilized to obtain the novel proteins and peptides of the invention. However, once these entities were prepared and their molecules sequenced, it is apparent to a person skilled in the art that the preferred method for making them would now be by chemical synthesis means. For example, there are available automated machines which can readily make proteins and peptides of the molecular sizes disclosed herein.

In the recombinant DNA procedures for making some of the proteins and peptides of the invention, an expression vector plasmid denoted pREV2.2 was used. This plasmid was initially constructed from a plasmid denoted pBG1

Plasmid pBG1 is deposited in the E. coli host MS371 with the Northern Regional Research Laboratory (NRRL, U.S. Department of Agriculture, Peoria, Illinois, USA. E. coli MS371(pBG1), NRRL B-15904, and E. coli MS371, NRRL B-15129, are disclosed in US-A-4271671. E. coli SG20251, NRRL B-15918, is disclosed in US-A-4691009.

E. coli JM103(pREV2.2), NRRL B-18091, and E. coli CAG629(pK1), NRRL B-18095, are disclosed in EP-A-0255190. This latter deposit can be subjected to standard techniques to separate the plasmid from the host cell, so that the host E. coli CAG629 can be used as disclosed herein.

This invention relates to twenty peptides. Each one of these peptides, given above, is an individual and distinct embodiment of this invention.

The novel HIV proteins and peptides can be expressed in Saccharomyces cerevisiae using plasmids containing the inducible galactose promoter from this organism, called YEp51 and YEp52; see Broach et al, Experimental Manipulation of Gene Expression (1983) p. 83, ed. M. Inouye, Academic Press. These plasmids contain the E. coli origin of replication, the gene for β-lactamase, the yeast LEU2 gene, the 2 μm origin of replication and the 2 μm cicle REP3 locus. Recombinant gene expression is driven by the yeast GAL10 gene promoter.

Yeast promoters such as galactose and alcohol dehydrogenase (Bennetzen, J.L. and Hall, B.D. [1982] J. Biol. Chem. 257:3018; Ammerer, G. in Methods in Enzymology [1983] Vol. 101, p. 192), phosphoglycerate kinase (Derynck, R., Hitzeman, R.A., Gray, P.W., Goeddel, D.V., in Experimental Manipulation of Gene Expression [1983] p. 247, ed. M. Inouye, Academic Press), triose phosphate isomerase (Alber, T. and Kawasaki, G. [1982] J. Molec. and Applied Genet. 1:419), or enolase (Innes, M.A. et al. [1985] Science 226:21) can be used.

The genes disclosed herein can be expressed in simian cells. When the genes encoding these proteins are cloned into one of the plasmids as described in Okayama and Berg (Okayama, H. and Berg, P. [1983] Molec. and Cell. Biol. 3:280) and references therein, or COS cells transformed with these plasmids, synthesis of HIV proteins can be detected immunologically.

Other mammalian cell gene expression/protein production systems can be used. Examples of other such systems are the vaccinia virus expression system (Moss, B. [1985] Immunology Today 6:243; Chakrabarti, S., Brechling, K., Moss, B. [1985] Molec. and Cell. Biol. 5:3403) and the vectors derived from murine retroviruses (Mulligan, R.C. in Experimental Manipulation of Gene Expression [1983] p. 155, ed. M. Inouye, Academic Press)

The HIV proteins and peptides of the subject invention can be chemically synthesized by solid phase peptide synthetic techniques such as BOC and FMOC (Merrifield, R.B. [1963] J. Amer. Chem. Soc. 85:2149; Chang, C. and Meienhofer, J. [1978] Int. J. Peptide Protein Res. 11:246).

As is well known in the art, the amino acid sequence of a protein is determined by the nucleotide sequence of the DNA. Because of the redundancy of the genetic code, i.e., more than one coding nucleotide triplet (codon) can be used for most of the amino acids used to make proteins, different nucleotide sequences can code for a particular amino acid. Thus, the genetic code can be depicted as follows:

Phenylalanine (Phe)    TTK
Leucine (Leu)    XTY
Isoleucine (Ile)    ATM
Methionine (Met)ATG
Valine (Val)    GTL
Serine (Ser)    QRS
Proline (Pro)    CCL
Threonine (Thr)    ACL
Alanine (Ala)    GCL
Tyrosine (Tyr)    TAK
Termination Signal    TAJ
Termination Signal    TGA
Histidine (His)    CAK
Glutamine (Gln)    CAJ
Asparagine (Asn)    AAK
Lysine (Lys)    AAJ
Aspartic acid (Asp)    GAK
Glutamic acid (Glu)    CAJ
Cysteine (Cys)    TGK
Tryptophan (Trp)    TGG
Arginine (Arg)    WGZ
Glycine (Gly)    GGL

Key: Each 3-letter deoxynucleotide triplet corresponds to a trinucleotide of mRNA, having a 5′-end on the left and a 3′-end on the right. All DNA sequences given herein are those of the strand whose sequence corresponds to the mRNA sequence, with thymine substituted for uracil. The letters stand for the purine or pyrimidine bases forming the deoxynucleotide sequence.

A = adenine
G = guanine
C = cytosine
T - thymine

EP 0 306 219 A2

X = T or C if Y is A or G
X = C if Y is C or T
Y = A, G, C or T if X is C
Y = A or G if X is T
W = C or A if Z is A or G
W = C if Z is C or T
Z = A, G, C or T if W is C
Z = A or G if W is A
QR = TC if S is A, G, C or T; alternatively QR = AG if S is T or C
J = A or G
K = T or C
L = A, T, C or G
M = A, C or T

The above shows that the novel amino acid sequences of the HIV proteins and peptides of the subject invention can be prepared by nucleotide sequences other than those disclosed herein. Functionally equivalent nucleotide sequences encoding the novel amino acid sequences of these HIV proteins and peptides, or fragments thereof having HIV antigenic or immunogenic or therapeutic activity, can be prepared by known synthetic procedures. Accordingly, the subject invention includes such functionally equivalent nucleotide sequences.

Thus the scope of the subject invention includes not only the specific nucleotide sequences depicted herein, but also all equivalent nucleotide sequences coding for molecules with substantially the same HIV antigenic or immunogenic or therapeutic activity.

Further, the scope of the subject invention is intended to cover not only the specific amino acid sequences disclosed, but also similar sequences coding for proteins or protein fragments having comparable ability to induce the formation of and/or bind to specific HIV antibodies.

The term "equivalent" is being used in its ordinary patent usage here as denoting a nucleotide sequence which performs substantially as the nucleotide sequence identified herein to produce molecules with substantially the same HIV antigenic or immunogenic or therapeutic activity in essentially the same kind of hosts. Within this definition are subfragments which have HIV antigenic or immunogenic or therapeutic activity.

As disclosed above, it is well within the skill of those in the genetic engineering art to use the nucleotide sequences encoding HIV antigenic or immunogenic or therapeutic activity of the subject invention to produce HIV proteins via microbial processes. Fusing the sequences into an expression vector and transforming or transfecting into hosts, either eukaryotic (yeast or mammalian cells) or prokaryotic (bacterial cells), are standard procedures used in producing other well-known proteins, e.g., insulin, interferons, human growth hormone, IL-1, IL-2, and the like. Similar procedures, or obvious modifications thereof, can be employed to prepare HIV proteins or peptides by microbial means or tissue-culture technology in accord with the subject invention.

The nucleotide sequences disclosed herein can be prepared by a "gene machine" by procedures well known in the art. This is possible because of the disclosure of the nucleotide sequence.

The restriction enzymes disclosed can be purchased from Bethesda Research Laboratories, Gaithersburg, MD, or New England Biolabs, Beverly, MA. The enzymes are used according to the instructions provided by the supplier.

The various methods employed in the preparation of the plasmids and transformation of host organisms are well known in the art. These procedures are all described in Maniatis, T., Fritsch, E.F., and Sambrook, J. (1982) Molecular Cloning: A Laboratory Manual, Cold Spring Harbor Laboratory, New York. Thus, it is within the skill of those in the genetic engineering art to extract DNA from microbial cells, perform restriction enzyme digestions, electrophorese DNA fragments, tail and anneal plasmid and insert DNA, ligate DNA, transform cells, e.g., E. coli cells, prepare plasmid DNA, electrophorese proteins, and sequence DNA.

Immunochemical assays employing the HIV proteins or peptides of the invention can take a variety of forms. The preferred type is a solid phase immunometric assay. In assays of this type, an HIV protein or peptide is immobilized on a solid phase to form an antigen-immunoadsorbent. The immunoadsorbent is incubated with the sample to be tested. After an appropriate incubation period, the immunoadsorbent is separated from the sample and labeled anti-(human IgG) antibody is used to detect human anti-HIV antibody bound to the immunoadsorbent. The amount of label associated with the immunoadsorbent can be compared to positive and negative controls to assess the presence or absence of anti-HIV antibody.

The immunoadsorbent can be prepared by adsorbing or coupling a purified HIV protein or peptide to a solid phase. Various solid phases can be used, such as beads formed of glass, polystyrene, polypropylene, dextran or other material. Other suitable solid phases include tubes or plates formed from or coated with these materials.

The HIV proteins or peptides can be either covalently or non-covalently bound to the solid phase by techniques such as covalent bonding via an amide or ester linkage or adsorption. After the HIV protein or peptide is affixed to the solid phase, the solid phase can be post-coated with an animal protein, e.g., 3% fish gelatin. This provides a blocking protein which reduces nonspecific adsorption of protein to the immunoadsorbent surface.

The immunoadsorbent is then incubated with the sample to be tested for anti-HIV antibody. In blood screening, blood plasma or serum is used. The plasma or serum is diluted with normal animal plasma or serum. The diluent plasma or serum is derived from the same animal species that is the source of the anti-(human IgG) antibody. The preferred anti-(human IgG) antibody is goat anti-(human IgG) antibody. Thus, in the preferred format, the diluent would be goat serum or plasma.

The conditions of incubation, e.g., pH and temperature, and the duration of incubation are not crucial These parameters can be optimized by routine experimentation. Generally, the incubation will be run for 1-2 hr at about 45°C in a buffer of pH 7-8.

After incubation, the immunoadsorbent and the sample are separated. Separation can be accomplished by any conventional separation technique such as sedimentation or centrifugation. The immunoadsorbent then may be washed free of sample to eliminate any interfering substances.

The immunoadsorbent is incubated with the labeled anti-(human IgG) antibody (tracer) to detect human antibody bound thereto. Generally the immunoadsorbent is incubated with a solution of the labeled anti-(human IgG) antibody which contains a small amount (about 1%) of the serum or plasma of the animal species which serves as the source of the anti-(human IgG) antibody. Anti-(human IgG) antibody can be obtained from any animal source. However, goat anti-(human IgG) antibody is preferred. The anti-(human IgG) antibody can be an antibody against the Fc fragment of human IgG, for example, goat anti-(human IgG), Fc antibody.

The anti-(human IgG) antibody or anti-(human IgG) Fc can be labeled with a radioactive material such as iodine; labeled with an optical label, such as a fluorescent material; or labeled with an enzyme such as horseradish peroxidase. The anti-human antibody can also be biotinylated and labeled avidin used to detect its binding to the immunoadsorbent.

After incubation with the labeled antibody, the immunoadsorbent is separated from the solution and the label associated with the immunoadsorbent is evaluated. Depending upon the choice of label, the evaluation can be done in a variety of ways. The label may be detected by a gamma counter if the label is a radioactive gamma emitter, or by a fluorimeter, if the label is a fluorescent material. In the case of an enzyme, label detection may be done colorimetrically employing a substrate for the enzyme.

The amount of label associated with the immunoadsorbent is compared with positive and negative controls in order to determine the presence of anti-HIV antibody. The controls are generally run concomitantly with the sample to be tested. A positive control is a serum containing antibody against HIV; a negative control is a serum from healthy individuals which does not contain antibody against HIV.

For convenience and standardization, reagents for the performance of the immunometric assay can be assembled in assay kits. A kit for screening blood, for example, can include:

(a) an immunoadsorbent, e.g., a polystyrene bead coated with an HIV protein or peptide;

(b) a diluent for the serum or plasma sample, e.g., normal goat serum or plasma;

(c) an anti-(human IgG) antibody, e.g., goat anti-(human IgG) antibody in buffered, aqueous solution containing about 1% goat serum or plasma;

(d) a positive control, e.g., serum containing antibody against at least one of the novel HIV proteins or peptides; and

(e) a negative control, e.g., pooled sera from healthy individuals which does not contain antibody against at least one of the novel HIV proteins or peptides.

If the label is an enzyme, an additional element of the kit can be the substrate for the enzyme.

Another type of assay for anti-HIV antibody is an antigen sandwich assay. In this assay, a labeled HIV protein or peptide is used in place of anti-(human IgG) antibody to detect anti-HIV antibody bound to the immunoadsorbent. The assay is based in principle on the bivalency of antibody molecules. One binding site of the antibody binds the antigen affixed to the solid phase; the second is available for binding the labeled antigen. The assay procedure is essentially the same as described for the immunometric assay except that after incubation with the sample, the immunodadsorbent in incubated with a solution of labeled HIV protein or peptide. HIV proteins or peptides can be labeled with radioisotope, an enzyme, etc. for this type of assay.

In a third format, the bacterial protein, protein A, which binds the Fc segment of an IgG molecule without interfering with the antigen-antibody interaction can be used as the labeled tracer to detect anti-HIV-antibody adsorbed to the immunoadsorbent. Protein A can be readily labeled with a radioisotope, enzyme or other detectable species.

Immunochemical assays employing an HIV protein or peptide have several advantages over those employing a whole (or disrupted) virus. Assays based upon an HIV protein or peptide will alleviate the concern over growing large quantities of infectious virus and the inherent variability associated with cell culturing and virus production. Further, the assay will help mitigate the real or perceived fear of contracting AIDS by technicians in hospitals, clinics and blood banks who perform the test.

Immunochemical assays employing recombinant envelope proteins from multiple viral variants have additional advantages over proteins from a single HIV variant. Assays incorporating protein sequences from multiple variants are more likely to accurately survey antibodies in the human population infected with diverse HIV variants. Also, solid phase enzyme-linked immunosorbent assay (ELISA) utilizing different HIV variant proteins would allow determination of prevalent serotypes in different geographic locations. This determination has not been possible until now as no available antibody detection kit utilizes more than one HIV variant.

Another use of recombinant proteins from HIV variants is to elicit variant-specific antisera in test animals. This antiserum would provide a reagent to identify which viral variant infected an individual. Currently. "virus typing" can only be done by viral gene cloning and sequencing. Binding of variant-specific serum to a patient viral isolate would provide a means of rapid detection not currently available. For example, sera raised to the proteins denoted PB1$_{IIIB}$, PB1$_{RF}$, PB1$_{MN}$, PB1$_{SC}$, and PB1$_{WMJ2}$ can be used to screen viral isolates from patients to determine which HIV variant the clinical isolate most closely resembles. This "screening" can be done by a variety of known antibody-antigen binding techniques.

Vaccines comprising one or more of the HIV proteins or peptides, disclosed herein, and variants thereof having antigenic properties, can be prepared by procedures well known in the art. For example, such vaccines can be prepared as injectables, e.g., liquid solutions or suspensions. Solid forms for solution in, or suspension in, a liquid prior to injection also can be prepared. Optionally, the preparation also can be emulsified. The active antigenic ingredient or ingredients can be mixed with excipients which are pharmaceutically acceptable and compatible with the active ingredient. Examples of suitable excipients are water, saline, dextrose, glycerol, ethanol, or the like, and combinations thereof. In addition, if desired, the vaccine can contain minor amounts of auxiliary substances such as wetting or emulsifying agents, pH buffering agents, or adjuvants such as aluminum hydroxide or muramyl dipeptide or variations thereof. In the case of peptides, coupling to larger molecules such as KLH sometimes enhances immunogenicity. The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers include, for example, polyalkalene glycols or triglycerides. Suppositories can be formed from mixtures containing the active ingredient in the range of about 0.5% to about 10%, preferably about 1 to about 2%. Oral formulations can include such normally employed excipients as, for example, pharmaceutical grades of mannitol, lactose, starch, magnesium stearate, sodium saccharide, cellulose, magnesium carbonate and the like. These compositions can take the form of solutions, suspensions, tablets, pills, capsules, sustained release formulations or powders and contain from about 10% to about 95% of active ingredient, preferably from about 25% to about 70%.

The proteins can be formulated into the vaccine as neutral or salt forms. Pharmaceutically acceptable salts include the acid addition salts (formed with the free amino groups of the peptide) and which are formed with inorganic acids such as, for example, hydrochloric or phosphoric acids, or such organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, capacity of the subject's immune system to synthesize antibodies, and the degree of protection desired. Precise amounts of active ingredient required to be administered depend on the judgment of the practitioner and are peculiar to each individual. However, suitable dosage ranges are of the order of about several hundred micrograms active ingredient per individual. Suitable regimes for initial administration and booster shots are also variable, but are typified by an initial administration followed in one or two week intervals by a subsequent injection or other administration.

HIV is known to undergo amino acid sequence variation, particularly in the envelope gene (Starcich,

7

B.R. [1986] Cell 45:637-648; Hahn, B.H. et al. [1986] Science 232:1548-1553). Over 100 variants have been analyzed by molecular cloning and restriction enzyme recognition analysis, and several of these have been analyzed by nucleotide sequencing. Some of these are the HIV isolates known as RF (Popovic, M. et al. [1984] Science 224:497-500), WMJ-1 (Hahn, B.H. et al [1986] Science 232:1548-1553), LAV (Wain-Hobson, S. et al. [1985] Cell 40:9-17), and ARV-2 (Sanchez-Pescador, R. et al. [1985] Science 227:484-492). The HIV proteins or peptides from different viral isolates can be used in vaccine preparations, as disclosed above, to protect against infections by different HIV isolates. Further, a vaccine preparation can be made using more than one recombinant antigenic protein from more than one HIV isolate to provide immunity and thus give better protection against AIDS.

The ability to modify peptides made by organic synthesis can be advantageous for diagnostic, therapeutic and prophylactic use by improving efficiency of immobilization, increasing protein stability, increasing immunogenicity, altering in immunogenicity, reducing toxicity, or allowing multiple variations simultaneously. For example, peptides can be modified to increase or decrease net charge by modification of amino or carboxyl groups (carbamylation, trifluoroacetylation or succinylation of amino groups; acetylation of carboxyl groups). Peptides can be made more stable by, for example, inclusion of D-amino acids or circularization of the peptide. Reductive state of peptides can be altered by, for example, sulfonation of cystinyl groups. The subject invention includes all such chemical modifications of the proteins and peptides disclosed herein so long as the modified protein and/or peptide retains substantially all the antigenic/immunogenic properties of the parent compound.

Peptides can also be modified to contain antigenic properties of more than one viral variant. This has been done, for example, with Foot and Mouth Disease virus.

Foot and Mouth Disease virus is similar to HIV in that multiple variants exist and immunization with one variant does not lead to protection against other variants. The real utility of peptides as immunogens is demonstrated by eliciting immunity to more than one variant by modification of the peptide to possess properties of both natural variants. When such a modified peptide was used to immunize test animals, they were protected against both Foot and Mouth virus strains A10 and A12 (Brown, F. in Virus Vaccines, ed. G. Dreesman, J. Bronson, R. Kennedy, pp. 49-54 [1985]).

Example 18 shows that a peptide containing amino acid sequences from two HIV variants can block virus neutralization activity of two virus specific neutralizing antisera. This suggests that a peptide or protein containing sequences of two or more HIV variants can elicit an immune response effective against two or more HIV variants.

Example 19 shows that co-immunization with envelope proteins from two HIV isolates elicits an immune response capable of neutralizing two HIV isolates. This suggests that co-immunization with proteins from two or more HIV variants can elicit an immune response effective against two or more HIV variants.

Following are examples which illustrate the process of the invention, including the best mode. These examples should not be construed as limiting. All solvent mixture proportions are by volume unless otherwise noted.

Example 1--Construction of plasmid pREV2.2

The pREV2.2 plasmid expression vector was constructed from plasmid pBG1. Plasmid pBG1 can be isolated from its E. coli host by well known procedures, e.g., using cleared lysate-isopycnic density gradient procedures, and the like. Like pBG1, pREV2.2 expresses inserted genes behind the E. coli promoter. The differences between pBG1 and pREV2.2 are the following:

1. pREV2.2 lacks a functional replication of plasmid (rop) protein.

2. pREV2.2 has the trpA transcription terminator inserted into the AatII site. This sequence insures transcription termination of over-expressed genes.

3. pREV2.2 has genes to provide resistance to ampicillin and chloramphenicol, whereas pBG1 provides resistance only to ampicillin.

4. pREV2.2 contains a sequence encoding sites for several restriction endonucleases.

The following procedures were used to make each of four changes listed above:

1a. 5 µg of plasmid pBG1 was restricted with NdeI, which gives two fragments of approximately 2160 and 3440 base pairs.

1b. 0.1 µg of DNA from the digestion mixture, after inactivation of the NdeI, was treated with T4 DNA ligase under conditions that favor intramolecular ligation (200 µl reaction volume using standard T4 ligase reaction conditions [New England Biolabs, Beverly, MA]). Intramolecular ligation of the 3440 base pair fragment gave an ampicillin resistant plasmid. The ligation mixture was transformed into the recipient strain E. coli JM103 (available from New England Biolabs) and ampicillin resistant clones were selected by standard procedures.

1c. The product plasmid, pBG1ΔN, where the 2160 base pair NdeI fragment is deleted from pBG1, was selected by preparing plasmid from ampicillin resistant clones and determining the restriction digestion patterns with NdeI and SalI (product fragments approximately 1790 and 1650). This deletion inactivates the rop gene that controls plasmid replication.

2a. 5 μg of pBG1ΔN was then digested with EcoRI and BclI and the larger fragment, approximately 2455 base pairs, was isolated.

2b. A synthetic double stranded fragment was prepared by the procedure of Itakura et al. (Itakura, K., Rossi, J.J. and Wallace, R.B. [1984] Ann. Rev. Biochem. 53:323-356, and references therein) with the following structure:

```
5'    GATCAAGCTTCTGCAGTCGACGCATGCGGATCCGGTACCCGGGAGCTCG    3'
3'    TTCGAAGACGTCAGCTGCGTACGCCTAGGCCATGGGCCCTCGAGCTTAA    5'
```

This fragment has BclI and EcoRI sticky ends and contains recognition sequences for several restriction endonucleases.

2c. 0.1 μg of the 2455 base pair EcoRI-BclI fragment and 0.01 μg of the synthetic fragment were joined with T4 DNA ligase and competent cells of strain JM103 were transformed. Cells harboring the recombinant plasmid, where the synthetic fragment was inserted into pBG1ΔN between the BclI and EcoRI sites, were selected by digestion of the plasmid with HpaI and EcoRI. The diagnostic fragment sizes are approximately 2355 and 200 base pairs. This plasmid is called pREV1.

2d. 5 μg of pREV1 were digested with AatII, which cleaves uniquely.

2e. The following double-stranded fragment was synthesized:

```
5'    CGGTACCAGCCCGCCTAATGAGCGGGCTTTTTTTTGACGT    3'
3'    TGCAGCCATGGTCGGGCGGATTACTCGCCCGAAAAAAAAC    5'
```

This fragment has AatII sticky ends and contains the trpA transcription termination sequence.

2f. 0.1 μg of AatII digested pREV1 was ligated with 0.01 μg of the synthetic fragment in a volume of 20 μl using T4 DNA ligase.

2g. Cells of strain JM103, made competent, were transformed and ampicillin resistant clones selected.

2h. Using a KpnI, EcoRI double restriction digest of plasmid isolated from selected colonies, a cell containing the correct construction was isolated. The sizes of the KpnI, EcoRI generated fragments are approximately 2475 and 80 base pairs. This plasmid is called pREV1TT and contains the trpA transcription terminator.

3a. 5 μg of pREV1TT, prepared as disclosed above (by standard methods) was cleaved with NdeI and XmnI and the approximately 850 base pair fragment was isolated.

3b. 5 μg of plasmid pBR325 (BRL, Gaithersburg, MD), which contains the genes conferring resistance to chloramphenicol as well as to ampicillin and tetracycline, was cleaved with BclI and the ends blunted with Klenow polymerase and deoxynucleotides. After inactivating the enzyme, the mixture was treated with NdeI and the approximately 3185 base pair fragment was isolated. This fragment contains the genes for chloramphenicol and ampicillin resistance and the origin of replication.

3c. 0.1 μ9 of the NdeI-XmnI fragment from pREV1TT and the NdeI-BclI fragment from pBR325 were ligated in 20 μl with T4 DNA ligase and the mixture used to transform competent cells of strain JM103. Cells resistant to both ampicillin and chloramphenicol were selected.

3d. Using an EcoRI and NdeI double digest of plasmid from selected clones, a plasmid was selected giving fragment sizes of approximately 2480, 1145, and 410 base pairs. This is called plasmid pREV1TT/chl and has genes for resistance to both ampicillin and chloramphenicol.

4a. The following double-stranded fragment was synthesized:

```
     MluI      EcoRV   ClaI BamHI   SalI HindIII SmaI

5' CGAACGCGTGGCCGATATCATCGATGGATCCGTCGACAAGCTTCCCGGGAGCT 3'
3' GCTTGCGCACCGGCTATAGTAGCTACCTAGGCAGCTGTTCGAAGGGCCC     5'
```

EP 0 306 219 A2

This fragment, with a blunt end and an Sstl sticky end, contains recognition sequences for several restriction enzyme sites.

4b. 5 μg of pREV1TT/chl was cleaved with NruI (which cleaves about 20 nucleotides from the BclI site) and SstI (which cleaves within the multiple cloning site). The larger fragment, approximately 3990 base pairs, was isolated from an agarose gel.

4c 0.1 μg of the NruI-SstI fragment from pREV1TT/chl and 0.01 μg of the synthetic fragment were treated with T4 DNA ligase in a volume of 20 μl.

4d. This mixture was transformed into strain JM103 and ampicillin resistant clones were selected.

4e. Plasmid was purified from several clones and screened by digestion with MluI or ClaI. Recombinant clones with the new multiple cloning site will give one fragment when digested with either of these enzymes, because each cleaves the plasmid once.

4f. The sequence of the multiple cloning site was verified. This was done by restricting the plasmid with HpaI and PvuII and isolating the 1395 base pair fragment, cloning it into the SmaI site of mp18 and sequencing it by dideoxynucleotide sequencing using standard methods.

4g. This plasmid is called pREV2.2.


Example 2--Construction of the bacterial expression vector REV2.1

Plasmid pREV2.1 was constructed using plasmid pREV2.2 and a synthetic oligonucleotide. The resulting plasmid was used to construct pPB1-Sub 1 and pPB1-Sub 2.

An example of how to construct pREV2.1 is as follows:

1. Plasmid pREV2.2 is cleaved with restriction enzymes NruI and BamHI and the 4 Kb fragment is isolated from an agarose gel.

2. The following double-strand oligonucleotide is synthesized:

5′    CGAACGCGTGGTCCGATATCATCGATG    3

3′    GCTTGCGCACCAGGCTATAGTAGCTACCTAG    5

3. The fragments from 1 and 2 are ligated in 20 μl using T4 DNA ligase, transformed into competent E. coli cells and chloramphenicol resistant colonies are isolated.

4. Plasmid clones are identified that contain the oligonucleotide from 2. spanning the region from the NruI site to the BamHI site and recreating these two restriction sites. This plasmid is termed pREV2.1.


Example 3--Construction of and expression from plasmid pPB1-Sub 1

Plasmid pPB1-Sub 1, which contains approximately 165 base pairs (bp) of DNA encoding essentially the HIV env gene from the PvuII site to the DraI site, and from which is synthesized an approximately 12 Kd fusion protein containing this portion of the gp120 envelope protein can be constructed as follows:

1. Restricting plasmid pPB1_IIIB with MluI and DraI and isolating the approximately 165 bp fragment.

2. Restricting plasmid pREV2.1 with MluI and SmaI and isolating the large fragment, approximately 4 Kd, from an agarose gel.

3. Ligating the fragment prepared in 2. with the pREV2.1 fragment in a volume of 20 μl using T4 DNA ligase, transforming the ligation mixture into competent cell strain CAG629, and selecting ampicillin-resistant transformants.

4. Selecting such transformants, by appropriate restriction patterns, that have the gp120 fragment cloned in the proper orientation to generate a fusion protein. When the strain harboring this recombinant plasmid is grown in 2% medium containing 50 μg/ml ampicillin and the total complement of cellular proteins are electrophoresed on a SDS-polyacrylamide gel, a protein of approximately 12 Kd can be visualized by either coomassie blue staining or by Western blot analysis using as probe selected sera from HIV infected individuals.


Example 4--Purification of recombinant protein containing HIV envelope sequences from plasmid pPB1-Sub 1

10

1. Growth of cells:

Cells were grown in a 10-1liter volume in a Chemap (Chemapec, Woodbury, NY) fermentor in 2% medium (22 yeast extract, bactotryptone, casamino acids [Difco, Detroit, MI], 0.2% potassium monobasic, 0.2% potassium dibasic, and 0.2% sodium dibasic). Fermentation temperature was 30°C, the pH was 6.8, and air was provided at 1 vvm. Plasmid selection was provided 20 μg/ml chloramphenicol. Typical cell yield (wet weight) was 30 g/l.

2. Cell Lysis:

50 g, wet cell weight, of E. coli containing the recombinant HIV envelope fusion protein were resuspended in a final volume of 100 ml in 50 mM Tris-Cl pH 8.0, 5 mM potassium ethylenediaminetetraacetic acid (KEDTA), 5 mM dithiothreitol (DTT), 15 mM β-mercaptoethanol, 0.5% TRITON® X-100 (Pharmacia, Piscataway, NJ), and 5 mM phenylmethylsulfonyl fluoride (PMSF). The suspension incubated for 30 min at room temperature.
This material was lysed using a BEAD-BEATERTM (Biospec Products, Bartlesville, OK) containing an equal volume of 0.1-0.15 mm glass beads. The lysis was done for 6 min at room temperature in 1-min intervals. The liquid was separated from the beads and centrifuged for 2.5 hr at 20,000 xg. The supernatant was removed and the pellet was resuspended in 100 ml 8 M urea, 20 mM Tris-Cl pH 8.0, 5 mM DTT, 15 mM β-mercaptoethanol, 5 mM PMSF, and 1 mM KEDTA. The pellet was solubilized using a polytron homogenizer and centrifuged at 20,000 xg for 2 hr.

3. CM Chromatography:

The dialysate was loaded onto a 100 ml column (2.5 cm x 20 cm) packed with CM FAST FLOW SEPHAROSE® (Pharmacia) equilibrated in 8 M urea, 10 mM 4-(2-hydroxyethyl-1-piperazine ethanesulfonic acid (HEPES) pH 6.5, 15 mM β-mercaptoethanol, and 1 mM KEDTA at room temperature. The column was washed with 200 ml equilibration buffer, and the protein eluted with a 1.0 liter linear gradient from 0-0.4 M NaCl. The HIV protein (12 Kd) eluted at approximately 0.2 M NaCl as assayed by SDS-polyacrylamide gel electrophoresis
Further purification was obtained by pooling Sub 1-containing fractions and applying to a S-200 (Pharmacia) gel filtration column equilibrated in the same buffer as the previous column.

Example 5--Construction of and expression from plasmid pPB1-Sub 2

Plasmid pPB1-Sub 2, which contains approximately 320 bp of DNA encoding essentially the HIV env gene from the Pvull site to the Scal site, and from which is synthesized an approximately 18 Kd fusion protein containing this portion of the gp120 envelope protein, can be constructed as follows:
1. Restricting the pPB1IIIB plasmid with Mlu1 and Scal and isolating the approximately 320 bp fragment.
2. Restricting plasmid pREV2.1 with Mlul and Smal and isolating the large fragment, approximately 4 Kd, from an agarose gel.
3. Ligating the fragment prepared in 2. with the pREV2.1 fragment in a volume of 20 μl using T4 DNA ligase, transforming the ligation mixture into competent cell strain SG20251 and selecting ampicillin-resistant transformants.
4. Selecting such transformants, by appropriate restriction patterns, that have the gp120 fragment cloned in the proper orientation to generate a fusion protein. When the strain harboring this recombinant plasmid is grown in 2% medium containing 50 μg/ml ampicillin and the total complement of cellular proteins electrophoresed on an SDS-polyacrylamide gel, a protein of approximately 18 Kd can be visualized by either coomassie blue staining or by Western blot analysis using as probe selected sera from HIV infected individuals.

Example 6--Purification of recombinant protein containing HIV envelope sequences from plasmid pPB1-Sub 2

1. Growth of cells:

Cells were grown in a 10-1iter volume in a Chemap fermentor in 2% medium. Fermentation temperature was 37° C, the pH was 6.8, and air was provided at 1 vvm. Plasmid selection was provided by 20 μg/ml chloramphenicol. Typical cell yield (wet weight) was 30 g/l.

2. Cell lysis:

50 g, wet cell weight, of E. coli containing the recombinant HIV envelope fusion protein were resuspended in a final volume of 100 ml in 50 mM Tris-Cl pH 8.0, 5 m KEDTA, 5 mM DTT, 15 mM β-mercaptoethanol, 0.5% TRITONTM X-100, and 5 mM PMSF. The suspension incubated for 30 min at room temperature.

This material was lysed using a BEAD-BEATERTM containing an equal volume of 0.1-0.15 mm glass beads. The lysis was done for 6 min at room temperature in 1 min intervals. The liquid was separated from the beads and centrifuged for 2.5 hr at 20,000 xg. The supernatant was removed and the pellet was resuspended in 100 ml 6 M guanidine-hydrochloride, 20 mM Tris-Cl pH 8.0, 5 mM DTT, 15 mM β-mercaptoethanol, 5 mM PMSF, and 1 mM KEDTA. The pellet was solubilized using a polytron homogenizer and centrifuged at 20,000 xg for 2 hr.

The supernate (90 ml) was dialyzed against 4 liters of 8 M urea, 20 mM sodium formate, pH 4.0, 1 mM EDTA, and 15 mM β-mercaptoethanol. Dialysis was done each time for 8 hr or longer with three changes of buffer. Spectraphor dialysis tubing (S/P, McGraw Park, IL) with a 3.5 Kd MW cut-off was used.

3. CM Chromatography--

The dialysate was loaded onto a 100 ml column (2.5 cm x 20 cm) packed with CM FAST FLOW SEPHAROSE® (Pharmacia) equilibrated in 8 M urea, 20 mM sodium formate pH 4.0, 15 mM β-mercaptoethanol, and 1 mM Na EDTA at room temperature. The column was washed with 200 ml equilibration buffer, and the protein eluted with a 1.0 liter linear gradient from 0-0.4 M NaCl. The HIV protein (18 Kd) eluted at approximately 0.2 M NaCl as assayed by SDS-polyacrylamide gel electrophoresis.

Further purification was obtained by pooling Sub 2-containing fractions and applying to a S-200 (Pharmacia) gel filtration column equilibrated in the same buffer as the previous column.

Example 7--Cleavage of Sub 2 to yield CnBr1

CNBr1 protein containing 38 amino acids of HIV sequences was obtained by chemical cleavage of purified Sub 2 protein. CNBr cleavage of proteins is a well-known procedure in the art. This specific cleavage was carried out as follows: Sub 2 protein, which contains one internal methionine, at a concentration of 5 mg/ml, was mixed with 702 formic acid and CNBR added at a ratio of 150 moles CNBr per mole of methionine. The reaction was allowed to go overnight at room temperature in the absence of air. The sample was lyophilized and purified by high pressure liquid chromatography (HPLC). A C18 column was used with an eluant of acetonitrile, containing 0.1% trifluoroacetic acid Protein CNBr1, corresponding to the N-terminus of Sub 2, was isolated by standard procedures.

Example 8--Synthetic peptides

A number of synthetic peptides listed in Tables 9 through 14 were isolated. Peptide 138 is a hybrid of $HIV_{IIIB}$ and $HIV_{RF}$ envelope sequences. Synthesis of peptides can be done by a variety of established procedures, for example, automated peptide synthesis. The product of synthesis can be further purified by a number of established separatory techniques, for example, reverse phase chromatography.

Example 9--Construction of and expression from plasmid pPB1<sub>RF</sub>

Plasmid pPB1$_{RF}$, which contains approximately 565 bp of DNA encoding essentially the HIV$_{RF}$ env gene from the PvuII site to the BglII site, and from which is synthesized an approximately 27 Kd fusion protein containing this portion of the gp120 envelope protein can be constructed as follows:

1. Synthesizing DNA fragment in Table 4A.

2. Restricting plasmid pREV2.2 with EcoRV and BamHI and isolating the large fragment, approximately 4 Kd, from an agarose gel.

3. Ligating the fragment prepared in 1. with the pREV2.2 fragment in a volume of 20 μl using T4 DNA ligase, transforming the ligation mixture into competent cell strain CAG 629, and selecting ampicillin-resistant transformants.

4 Selecting such transformants, by appropriate restriction patterns, that have the gp120 fragment cloned in the proper orientation to generate a fusion protein. When the strain harboring this recombinant plasmid is grown at 32°C in 2% medium containing 50 μg/ml ampicillin and the total complement of cellular proteins electrophoresed on an SDS-polyacrylamide gel, a protein of approximately 27 Kd can be visualized by either coomassie blue staining or by Western blot analysis using as probe selected sera from HIV infected individuals.

Example 10--Purification of recombinant protein containing HIV envelope sequences from plasmid pPB1<sub>RF</sub>

1. Growth of cells:

Cells were grown in a 10-1iter volume in a Chemap fermentor in 2% medium. Fermentation temperature was 30°C, the pH was 6.8, and air was provided at 1 vvm. Plasmid selection was provided by 20 μg/ml chloramphenicol. Typical cell yield (wet weight) was 30 g/l.

2. Cell lysis:

50 g, wet cell weight, of E. coli containing the recombinant HIV envelope fusion protein were resuspended in a final volume of 100 ml in 50 mM Tris-Cl pH 8.0, 5 mM KEDTA, 5 mM DDT, 15 mM β-mercaptoethanol, 0.5% TRITON® X-100, and 5 mM PMSF. 300 mg lysozyme was added and the suspension incubated for 30 min at room temperature.

This material was lysed using a BEAD-BEATERTM containing an equal volume of 0.1-0.15 mm glass beads. The lysis was done for 6 min at room temperature in 1 min intervals. The liquid was separated from the beads and centrifuged for 2.5 hr at 20,000 xg. The supernatant was removed and the pellet was resuspended in 100 ml 8 M urea, 20 mM Tris-Cl pH 8.0, 5 mM DT, 15 mM β-mercaptoethanol, 5 mM PMSF, and 1 mM KEDTA. The pellet was solubilized using a polytron homogenizer and centrifuged at 20,000 xg for 2 hr.

The supernate (90 ml) was dialysed against 4 liters of 8 M urea, 20 mM HEPES, pH 6.5, 1 mM EDTA, and 15 mM 5-mercaptoethanol. Dialysis was done each time for 8 hr or longer with three changes of buffer. Spectraphor dialysis tubing with a 3.5 Kd MW cut-off was used.

3. CM Chromatography:

The dialysate was loaded onto a 100 ml column (2.5 cm x 20 cm) packed with CM FAST FLOW SEPHAROSE® equilibrated in 8 M urea, 10 mM HEPES pH 6.5, 15 mM β-mercaptoethanol, and 1 mM Na EDTA at room temperature. The column was washed with 200 ml equilibration buffer, and the protein eluted with a 1.0 liter linear gradient from 0-0.4 M NaCl. The HIV protein (26 Kd) eluted at approximately 0.2 M NaCl as assayed by SDS-polyacrylamide gel electrophoresis.

Further purification was obtained by pooling PB1$_{RF}$-containing fractions and applying to a S-300 gel filtration column equilibrated in the same buffer as the previous column.

Example 11--Construction of and expression from plasmid pPB1$_{MN}$

Plasmid pPB1$_{MN}$, which contains approximately 600 bp of DNA encoding essentially the HIV$_{MN}$ env gene from the BglII site to the BglII site, and from which is synthesized an approximately 28 Kd fusion protein containing this portion of the gp120 envelope protein, can be constructed as follows:

1. Synthesizing DNA fragment in Table 5A.
2. Restricting plasmid pREV2.2 with BamHI.
3. Ligating the fragment prepared in 1. with the pREV2.2 fragment in a volume of 20 μl using T4 DNA ligase, transforming the ligation mixture into competent cell strain CAG629, and selecting ampicillin-resistant transformants.
4. Selecting such transformants, by appropriate restriction patterns, that have the gp120 fragment cloned in the proper orientation to generate a fusion protein. When the strain harboring this recombinant plasmid is grown at 32°C in 2% medium containing 50 μg/ml ampicillin and the total complement of cellular proteins electrophoresed on an SDS-polyacrylamide gel, a protein of approximately 28 Kd can be visualized by either coomassie blue staining or by Western blot analysis using as probe selected sera from HIV infected individuals.

Example 12--Purification of recombinant protein containing HIV envelope sequences from plasmid pPB1$_{MN}$

1. Growth of cells:

Cells were grown in a 10-liter volume in a Chemap fermentor in 2% medium. Fermentation temperature was 30°C, the pH was 6.8, and air was provided at 1 vvm. Plasmid selection was provided by 20 μg/ml chloramphenicol. Typical cell yield (wet weight) was 30 g/l.

2. Cell lysis:

50 g, wet cell weight, of E. coli containing the recombinant HIV envelope fusion protein were resuspended in a final volume of 100 ml in 50 mM Tris-Cl pH 8.0, 5 mM KEDTA, 5 mM DTT, 15 mM β-mercaptoethanol, 0 5% TRITON® X-100, and 5 mM PMSF. 300 mg lysozyme was added and the suspension incubated for 30 min at room temperature.

This material was lysed using a BEAD-BEATER™ containing an equal volume of 0.1-0.15 mm glass beads. The lysis was done for 6 min at room temperature in 1 min intervals. The liquid was separated from the beads and centrifuged for 2.5 hr at 20,000 xg. The supernatant was removed and the pellet was resuspended in 100 ml 8 M urea, 20 mM Tris-Cl pH 8.0, 5 mM DTT, 15 mM β-mercapoethanol, 5 mM PMSF, and 1 mM KEDTA. The pellet was solubilized using a polytron homogenizer and centrifuged at 20,000 xg for 2 hr.

The supernate (90 ml) was dialysed against 4 liters of 8 M urea, 20 mM HEPES, pH 6.5, 1 mM EDTA, and 15 mM β-mercaptoethanol. Dialysis was done each time for 8 hr or longer with three changes of buffer. Spectraphor dialysis tubing with a 3.5 Kd MW cut-off was used.

3. CM Chromatography:

The dialysate was loaded onto a 100 ml column (2.5 cm x 20 cm) packed with CM FAST FLOW SEPHAROSE® equilibrated in 8 M urea, 10 mM HEPES pH 6.5, 15 mM β-mercaptoethanol, and 1 mM KEDTA at room temperature. The column was washed with 200 ml equilibration buffer, and the protein eluted with a 1.0 liter linear gradient from 0-0.4 M NaCl. The HIV protein (28 Kd) eluted at approximately 0.2 M NaCl as assayed by SDS-polyacrylamide gel electrophoresis.

Further purification was obtained by pooling PB1$_{MN}$-containing fractions and applying to a S-300 gel filtration column equilibrated in the same buffer as the previous column.

Example 13--Construction of and expression from plasmid pPB1<sub>SC</sub>

Plasmid pPB1<sub>SC</sub>, which contains approximately 570 bp of DNA encoding essentially the HIV<sub>SC</sub> env gene from the PvuII site to the BgIII site, and from which is synthesized an approximately 26 Kd fusion protein containing this portion of the gp120 envelope protein can be constructed as follows:

1. Synthesizing DNA fragment in Table 6A.

2. Restricting plasmid pREV2.2 with EcoRV and BamHI and isolating the large fragment, approximately 4 Kd, from an agarose gel.

3. Ligating the fragment prepared in 1. with the pREV2.2 fragment in a volume of 20 µl using T4 DNA ligase, transforming the ligation mixture into competent cell strain CAG 629, and selecting ampicillin-resistant transformants.

4. Selecting such transformants, by appropriate restriction patterns, that have the gp120 fragment cloned in the proper orientation to generate a fusion protein. When the strain harboring this recombinant plasmid is grown at 32°C in 2% medium containing 50 µg/ml ampicillin and the total complement of cellular proteins electrophoresed on an SDS-polyacrylamide gel, a protein of approximately 26 Kd can be visualized by either coomassie blue staining or by Western blot analysis using as probe selected sera from HIV infected individuals.

Example 14--Purification of recombinant protein containing HIV envelope sequences from plasmid pPB1<sub>SC</sub>

1. Growth of cells:

Cells were grown in a 10-liter volume in a Chemap fermentor in 2% medium. Fermentation temperature was 30°C, the pH was 6.8, and air was provided at 1 vvm. Plasmid selection was provided by 20 µg/ml chloramphenicol. Typical cell yield (wet weight) was 30 g/l.

Cell lysis:

50 g, wet cell weight, of E. coli containing the recombinant HIV envelope fusion protein were resuspended in a final volume of 100 ml in 50 mM Tris-Cl pH 8.0, 5 mM KEDTA, 5 mM DTT, 15 mM β-mercaptoethanol, 0.5% TRITON X-100, and 5 mM PMSF. 300 mg lysozyme was added and the suspension incubated for 30 min at room temperature.

This material was lysed using a BEAD-BEATER™ containing an equal volume of 0.1-0.15 mm glass beads. The lysis was done for 6 min at room temperature in 1 min intervals. The liquid was separated from the beads and centrifuged for 2.5 hr at 20,000 xg. The supernatant was removed and the pellet was resuspended in 100 ml 8 M urea, 20 mM Tris-Cl pH 8.0, 5 mM DTT, 15 mM β-mercaptoethanol, 5 mM PMSF, and 1 mM KEDTA. The pellet was solubilized using a polytron homogenizer and centrifuged at 20,000 xg for 2 hr.

The supernate (90 ml) was dialysed against 4 liters of 8 M urea, 20 mM HEPES, pH 6.5, 1 mM EDTA, and 15 mM β-mercaptoethanol, and 1 mM KEDTA at room temperature. The dialysate was loaded onto a 100 ml column (2.5 cm x 20 cm) packed with CM FAST FLOW SEPHAROSE® equilibrated in 8 M urea, 10 mM HEPES pH 6.5, 15 mM β-mercaptoethanol, and 1 mM KEDTA at room temperature. The column was washed with 200 ml equilibration buffer, and the protein eluted with a 1.0 liter linear gradient from 0-0.4 M NaCl. The HIV protein (26 Kd) eluted at approximately 0.2 M NaCl as assayed by SDS-polyacrylamide gel electrophoresis.

Further purification was obtained by pooling pPB1<sub>SC</sub>-containing fractions and applying to a S-300 gel filtration column equilibrated in the same buffer as the previous column.

Example 15--Construction of and expression from plasmid pPB1<sub>WMJ2</sub>

Plasmid pPB1<sub>WMJ2</sub>, which contains approximately 560 bp of DNA encoding essentially the HIV<sub>WMJ2</sub> env gene and from which is synthesized an approximately 26 Kd fusion protein containing this portion of the gp120 envelope protein can be constructed as follows:

1. Synthesizing DNA fragment in Table 7A.

2. Restricting plasmid pREV2.2 with EcoRV and BamHI and isolating the large fragment, approximately 4 Kd, from an agarose gel.

3. Ligating the fragment prepared in 1. with the pREV2.2 fragment in a volume of 20 μl using T4 DNA ligase, transforming the ligation mixture into competent cell strain $CAG_{629}$, and selecting ampicillin-resistant transformants.

4. Selecting such transformants, by appropriate restriction patterns, that have the gp120 fragment cloned in the proper orientation to generate a fusion protein. When the strain harboring this recombinant plasmid is grown at 32°C in 2% medium containing 50 μg/ml ampicillin and the total complement of cellular proteins electrophoresed on an SDS-polyacrylamide gel, a protein of approximately 26 Kd can be visualized by either coomassie blue staining or by Western blot analysis using as probe selected sera from HIV infected individuals.

Example 16--Purification of recombinant protein containing HIV envelope sequences from plasmid $pPB1_{WMJ2}$

1. Growth of cells:

Cells were grown in a 10-1liter volume in a Chemap fermentor in 2% medium. Fermentation temperature was 30°C, the pH was 6.8, and air was provided at 1 vvm. Plasmid selection was provided by 20 μg/ml chloramphenicol. Typical cell yield (wet weight) was 30 g/l.

2. Cell lysis:

50 g, wet cell weight, of E. coli containing the recombinant HIV envelope fusion protein were resuspended in a final volume of 100 ml in 50 mM Tris-Cl pH 8.0, 5 mM KEDTA, 5 mM DTT, 15 mM β-mercaptoethanol, 0.5% TRITON® X-100, and 5 mM PMSF. 300 mg lysozyme was added and the suspension incubated for 30 min at room temperature.

This material was lysed using a BEAD-BEATER™ containing an equal volume of 0.1-0.15 mm glass beads. The lysis was done for 6 min at room temperature in 1 min intervals. The liquid was separated from the beads and centrifuged for 2.5 hr at 20,000 xg. The supernatant was removed and the pellet was resuspended in 100 ml 8 M urea, 20 m Tris-Cl pH 8.0, 5 mM DTT, 15 mM β-mercaptoethanol, 5 mM PMSF, and 1 mM KEDTA. The pellet was solubilized using a polytron homogenizer and centrifuged at 20,000 xg for 2 hr.

The supernate (90 ml) was dialysed against 4 liters of 8 M urea, 20 mM HEPES, pH 6.5, 1 mM EDTA, and 15 mM β-mercaptoethanol. Dialysis was done each time for 8 hr or longer with three changes of buffer. Spectraphor dialysis tubing with a 3.5 Kd MW cut-off was used.

3. CM Chromatography:

The dialysate was loaded onto a 100 ml column (2.5 cm x 20 cm) packed with CM FAST FLOW SEPHAROSE® equilibrated in 8 M urea, 10 mM HEPES pH 6.5, 15 mM β-mercaptoethanol, and 1 mM Na EDTA at room temperature. The column was washed with 200 ml equilibration buffer, and the protein eluted with a 1.0 liter linear gradient from 0-0.4 M NaCl. The HIV protein (26 Kd) eluted at approximately 0.2 M NaCl as assayed by SDS-polyacrylamide gel electrophoresis.

Further purification was obtained by pooling $PB1_{WMJ2}$-containing fractions and applying to a S-300 gel filtration column equilibrated in the same buffer as the previous column.

Example 17--Cell fusion inhibition

The in vitro fusion of HIV infected cells with T4 positive T cells is measured in the presence and absence of immune serum. This is a well-known assay (Putney, et al. [1986] Science 234:1392-1395).

Chronically infected cells and uninfected cells (1:15) are mixed and incubated 24 hr. Foci of fused cells (giant cells) are then counted (usually about 60). Dilution of an immune serum, for example, serum to the entire HIV envelope (gp160) or to a protein or peptide of the invention, is added when the cells are mixed. A 90% decrease in giant cells after 24 hr indicates the immune serum can block fusion. This assay can be done with cells infected with various virus strains, for example, HIV$_B$ and HIV$_{RF}$.

Example 18--Competition cell fusion

Using the assay described in Example 17, one can determine if proteins or peptides contain the epitope recognized by antibodies that are responsible for cell fusion inhibition. For example, fusion inhibition of HIV$_{IIIB}$ infected cells by antiserum to PB1-III$_B$ protein is abated by addition of PB1 protein to 5 $\mu$g/ml. Using antiserum to PB1 and adding any one of the proteins or peptides, for example, Sub 2, Sub 1, CNBr1, peptide 135 or peptide 136 at 5 $\mu$g/ml totally blocks the activity of the PB1 antiserum. Peptide 138 at a concentration of greater than 20 $\mu$g/ml prevents inhibition of fusion by antisera to PB1$_{RF}$ of HIV$_{RF}$-infected cells. Similarly, at a concentration of 10 $\mu$g/ml, peptide 138 completely prevents inhibition of fusion by antisera to PB1-III$_B$ of HIV$_{IIIB}$-infected cells. This peptide, which is a hybrid between III$_B$ and $_{RF}$ sequences thus has activity affecting both viral isolates.

Example 19--Co-immunization of PB1-III$_B$ and PB1$_{RF}$

Antisera from an animal immunized with two PB1 proteins from HIV$_{IIIB}$ and HIV$_{RF}$ isolates were capable of blocking cell fusion of both HIV$_{IIIB}$- and HIV$_{RF}$-infected cells. This demonstrates that co-immunization with separate proteins containing envelope sequences of two HIV isolates elicits an immune response capable of neutralizing both isolates. This novel property of small proteins or peptides blocking immune serum has not been described before.

Some of the proteins and peptides of the subject invention contain the entire epitope for raising humoral immune responses that neutralize HIV infection and block HIV infected cell fusion. This is shown by these proteins and peptides competing these activities out of serum from animals immunized with the entire HIV envelope protein, or the PB1 protein portion of the HIV envelope. More specifically, proteins and peptides that can compete these activities from anti-gp160 or anti-PB1 sera are Sub 2, Sub 1, CNBr1, peptide 135, and peptide 136.

The proteins and peptides of the invention also can be used to stimulate a lymphocyte proliferative response in HIV infected humans. This then would stimulate the immune system to respond to HIV in such individuals.

Example 20--Construction of and expression from plasmid pPB1$_{IIIB}$

Plasmid pPB1, which contains approximately 540 base pairs of DNA encoding essentially the HIV env gene from the PvuII site to the BglII site, and from which is synthesized an approximately 26 kD fusion protein containing this portion of the gp120 envelope protein can be constructed as follows:

1. Synthesizing the DNA with the sequence shown in Table 15: This DNA fragment can be synthesized by standard methods and encodes a portion of gp120 It has a blunt end on the 5′ end and an end which will ligate with a BamHI overhang on the 3′ end.

2. Restricting 5 $\mu$g plasmid pREV2.2 with EcoRV and BamHI and isolating the large fragment, approximately 4 kD, from an agarose gel.

3. Ligating 0.1 $\mu$g of the fragment in Table 15 with 0.1 $\mu$g of the pREV2.2 fragment in a volume of 20 $\mu$l using T4 DNA ligase, transforming the ligation mixture into competent cell strain SG20251, and selecting ampicillin resistant transformants.

4. Using the AhaIII restriction pattern of purified plasmid, selecting cells harboring the recombinant plasmid with the synthesized fragment in the orientation whereby the fragment blunt end ligated to the REV2.2 EcoRV end and the BamHI overhanging ends ligated together. AhaIII digestion of the proper plasmid gives fragment lengths of approximately 1210, 1020, 750, 690, 500, 340, and 20 base pairs. When the strain harboring this recombinant plasmid is grown in 2% medium containing 50 $\mu$g/ml ampicillin and the total complement of cellular proteins electrophoresed on an SDS-polyacrylamide gel, a protein of approximately 26 kD can be visualized by either coomassie blue staining or by western blot analysis using as probe selected sera from AIDS, ARC, or HIV infected individuals.

Example 21--Purification of recombinant protein containing HIV envelope sequences from plasmid pPB1$_{IIIB}$

1. Growth of cells:

Cells were grown in a 10 liter volume in a Chemap fermentor in 2% medium. Fermentation temperature was 37° C, the pH was 6.8, and air was provided at 1 vvm. Plasmid selection was provided by 50 $\mu$g/ml ampicillin and 20 $\mu$g/ml chloramphenicol. Typical cell yield (wet weight) was 30 g/l.

2. Cell Lysis:

50 g. wet cell weight, of E. coli containing the recombinant HIV envelope fusion protein were resuspended in a final volume of 100 ml in 50 mM Tris-Cl pH 8.0, 5 mM KEDTA, 5 mM DTT, 15 mM $\beta$-mercaptoethanol, 0 5% Triton X-100, and 5 mM PMSF. 300 mg lysozyme was added and the suspension incubated for 30 min at room temperature.

This material was lysed using a BEAD-BEATERTM (Biospec Products, Bartlesville, OK) containing an equal volume of 0.1-0.15 $\mu$m glass beads. The lysis was done for 6 min at room temperature in 1 min intervals. The liquid was separated from the beads and centrifuged for 2.5 hr at 20,000 xg. The supernatant was removed and the pellet was resuspended in 100 ml 6 M guanidine-hydrochloride, 20 mM Tris-Cl pH 8.0, 5 mM DTT, 15 mM $\beta$-mercaptoethanol, 5 mM PMSF, and 1 mM KEDTA. The pellet was solubilized using a polytron homogenizer and centrifuged at 20,000 xg for 2 hr.

The supernate (90 ml) was dialysed against 4 liters of 8 M urea, 20 mM Tris-Cl, pH 8.0, 1 mM EDTA, and 15 mM $\beta$-mercaptoethanol. Dialysis was done each time for 8 hr or longer with three changes of buffer. Spectraphor dialysis tubing (S/P, McGraw Park, IL) with a 3.5 kD MW cut-off was used.

3. CM chromatography

The dialysate was loaded onto a 550 ml column (5 cm x 28 cm) packed with CM Fast Flow Sepharose (Pharmacia), equilibrated in 8 M urea, 10 mM potassium phosphate pH 7.0, 15 mM $\beta$-mercaptoethanol, and 1 mM KEDTA at room temperature. The column was washed with 2 liters equilibration buffer, and the protein eluted with a 5.0 liter linear gradient from 0-0.4 M NaCl. The HIV protein (26 kD) eluted at approximately 0.2 M NaCl as assayed by SDS-polyacrylamide gel electrophoresis.

This work was supported in part under contract number NO1-AI-62558, awarded to Repligen Corporation by the National Institute of Allergy and Infectious Diseases (NIAID).

## Table 1

### SYNTHETIC PEPTIDES OF THE
### N-TERMINAL REGION OF PB1

```
287                                                                                    325
E R V A D L N Q S V E I N C T R P N N N T R K S I R I Q R G P G R A F V T I G K I G N H R Q A N C Y
. . . . . . . . . |_____|
                                                                            PEPTIDE # 136

                          |                               |                                    |
                          Y C T R P N N N T R K S I R I Q R G P|G R A F V T I G K I G N H R Q A N C Y|
                              PEPTIDE # 131                       PEPTIDE # 132


E R V A D L N G S V E I N C T R P N N N T R K S I
        PEPTIDE # 134

                              N N T R K S I R I Q R G P G R A F V T I G K I G C
                                          PEPTIDE # 135
```

The one-letter symbol for the amino acids used in Table 1 is well known in the art. For convenience the relationship of the three-letter abbreviation and the one-letter symbol for amino acids is as follows: Ala = A, Arg = R, Asn = N, Asp = D, Cys = C, Gln = Q, Glu = E, Gly = G, His = H, Ile = I, Leu = L, Lys = K, Met = M, Phe = F, Pro = P, Ser = S, Thr = T, Trp = W, Tyr = Y, Val = V.

EP 0 306 219 A2

## TABLE 2

LeuAsnGlnSerValGluIleAsnCysThrArgProAsnAsnAsnThrArgLys
SerIleArgIleGlnArgGlyProGlyArgAlaPheValThrIleGlyLysIle
GlyAsnMetArgGlnAlaHisCysAsnIleSerArgAlaLysTrpAsnAsnThr

## TABLE 2A

CTGAACCAATCTGTAGAAATTAATTGTACAAGACCCAACAACAATACAAGAAAA
AGTATCCGTATCCAGAGAGGACCAGGGAGAGCATTTGTTACAATAGGAAAAATA
GGAAATATGAGACAAGCACATTGTAACATTAGTAGAGCAAAATGGAATAACACTTT

## TABLE 2B

MetLeuArgProValGluThrProThrArgGluIleLysLysLeuAspGlyLeu
TrpAlaPheSerLeuAspArgGluArgValAlaAspLeuAsnGlnSerValGlu
IleAsnCysThrArgProAsnAsnAsnThrArgLysSerIleArgIleGlnArg
GlyProGlyArgAlaPheValThrIleGlyLysIleGlyAsnMetArgGlnAla
HisCysAsnIleSerArgAlaLysTrpAsnAsnThrLeuGlyAlaArgIleLeu
GluAspGluArgAlaSer

## TABLE 2C

ATGTTACGTCCTGTAGAAACCCCAACCCGTGAAATCAAAAAACTGGACGGCCTG
TGGGCATTCAGTCTGGATCGCGAACGCGTGGCCGATCTGAACCAATCTGTAGAA
ATTAATTGTACAAGACCCAACAACAATACAAGAAAAAGTATCCGTATCCAGAGA
GGACCAGGGAGAGCATTTGTTACAATAGGAAAAATAGGAAATATGAGACAAGCA
CATTGTAACATTAGTAGAGCAAAATGGAATAACACTTTGGGAGCTCGAATTCTT
GAAGACGAAAGGGCCTCG

## TABLE 3

LeuAsnGlnSerValGluIleAsnCysThrArgProAsnAsnAsnThrArgLys
SerIleArgIleGlnArgGlyProGlyArgAlaPheValThrIleGlyLysIle
GlyAsnMetArgGlnAlaHisCysAsnIleSerArgAlaLysTrpAsnAsnThr
LeuLysGlnIleAspSerLysLeuArgGluGlnPheGlyAsnAsnLysThrIle
IlePheLysGlnSerSerGlyGlyAspProGluIleValThrHisSerPheAsn
CysGlyGlyGluPhePheTyrCysAsnSerThrGlnLeuPheAsnSer

## TABLE 3A

CTGAACCAATCTGTAGAAATTAATTGTACAAGACCCAACAACAATACAAGAAAA
AGTATCCGTATCCAGAGAGGACCAGGGAGAGCATTTGTTACAATAGGAAAAATA
GGAAATATGAGACAAGCACATTGTAACATTAGTAGAGCAAAATGGAATAACACT
TTAAAACAGATAGATAGCAAATTAAGAGAACAATTTGGAAATAATAAAACAATA
ATCTTTAAGCAGTCCTCAGGAGGGGACCCAGAAATTGTAACGCACAGTTTTAAT
TGTGGAGGGGAATTTTTTCTACTGTAATTCAACACAACTGTTTAATAGT

20

### TABLE 3B

MetLeuArgProValGluThrProThrArgGluIleLysLysLeuAspGlyLeu
TrpAlaPheSerLeuAspArgGluArgValAlaAspLeuAsnGlnSerValGlu
IleAsnCysThrArgProAsnAsnAsnThrArgLysSerIleArgIleGlnArg
GlyProGlyArgAlaPheValThrIleGlyLysIleGlyAsnMetArgGlnAla
HisCysAsnIleSerArgAlaLysTrpAsnAsnThrLeuLysGlnIleAspSer
LysLeuArgGluGlnPheGlyAsnAsnLysThrIleIlePheLysGlnSerSer
GlyGlyAspProGluIleValThrHisSerPheAsnCysGlyGlyGluPhePhe
TyrCysAsnSerThrGlnLeuPheAsnSerGlySerSerAsnSer

.

### TABLE 3C

ATGTTACGTCCTGTAGAAACCCCAACCCGTGAAATCAAAAAACTGGACGGCCTG
TGGGCATTCAGTCTGGATCGCGAACGCGTGGCCGATCTGAACCAATCTGTAGAA
ATTAATTGTACAAGACCCAACAACAATACAAGAAAAAGTATCCGTATCCAGAGA
GGACCAGGGAGAGCATTTGTTACAATAGGAAAAATAGGAAATATGAGACAAGCA
CATTGTAACATTAGTAGAGCAAAATGGAATAACACTTTAAAACAGATAGATAGC
AAATTAAGAGAACAATTTGGAAATAATAAAACAATAATCTTTAAGCAGTCCTCA
GGAGGGGACCCAGAAATTGTAACGCACAGTTTTAATTGTGGAGGGGAATTTTTC
TACTGTAATTCAACACAACTGTTTAATAGTGGGAGCTCGAATTCT

### TABLE 4

LeuAsnAlaSerValGlnIleAsnCysThrArgProAsnAsnAsnThrArgLys
SerIleThrLysGlyProGlyArgValIleTyrAlaThrGlyGlnIleIleIleGly
AspIleArgLysAlaHisCysAsnLeuSerArgAlaGlnTrpAsnAsnThrLeu
LysGlnValValThrLysLeuArgGluGlnPheAspAsnLysThrIleValPhe
ThrSerSerSerGlyGlyAspProGluIleValLeuHisSerPheAsnCysGly
GlyGluPhePheTyrCysAsnThrThrGlnLeuPheAsnSerThrTrpAsnSer
ThrGluGlySerAsnAsnThrGlyGlyAsnAspThrIleThrLeuProCysArg
IleLysGlnIleValAsnMetTrpGlnGluValGlyLysAlaMetTyrAlaPro
ProIleSerGlyGlnIleLysCysIleSerAsnIleThrGlyLeuLeuLeuThr
ArgAspGlyGlyGluAspThrThrAsnThrThr

### TABLE 4A

CTGAATGCATCTGTACAAATTAATTGTACAAGACCCAACAACAATACAAGAAAA                    GACTTACG-
TAGACATGTTTAATTAACATGTTCTGGGTTGTTGTTATGTTCTTTT
AGTATAACTAAGGGACCAGGGAGAGTAATTTATGCAACAGGACAAATAATAGGA    TCATATTGATTCCCTG-
GTCCCTCTCATTAAATACGTTGTCCTGTTTATTATCCT
GATATAAGAAAAGCACATTGTAACCTTAGTAGAGCACAATGGAATAACACTTTA    CTATATTCTTTTCGTG-
TAACATTGGAATCATCTCGTGTTACCTTATTGTGAAAT
AAACAGGTAGTTACAAAATTAAGAGAACAATTTGACAATAAAACAATAGTCTTT    TTTGTCCATCAATGTTT-
TAATTCTCTTGTTAAACTGTTATTTTGTTATCAGAAA
ACGTCATCCTCAGGAGGGGACCCAGAAATTGTACTTCACAGTTTTAATTGTGGA    TGCAGTAGGAGTCCTC-
CCCTGGGTCTTTAACATGAAGTGTCAAAATTAACACCT
GGGGAATTTTTCTACTGTAATACAACACAACTGTTTAATAGTACTTGGAATAGT    CCCCTTAAAAAGAT-
GACATTATGTTGTGTTGACAAATTATCATGAACCTTATCA
ACTGAAGGGTCAAATAACACTGGAGGAAATGACACAATCACACTCCCATGCAGA    TGACTTCCCAGTTTAT-
TGTGACCTCCTTTACTGTGTTAGTGTGAGGGTACGTCT

ATAAAACAAATTGTAAACATGTGGCAGGAAGTAGGAAAAGCAATGTATGCCCCT TATTTTGTTTAACATTT-
GTACACCGTCCTTCATCCTTTTCGTTACATACGGGGA
CCCATCAGTGGACAAATTAAATGTATATCAAATATTACAGGGCTACTATTAACA GGGTAGTCACCTGTT-
TAATTTACATATAGTTTATAATGTCCCGATGATAATTGT
AGAGATGGGGGTGAAGATACAACTAATACTACAGA TCTCTACCCCCACTTCTATGTTGATTATGATGTCTC-
TAG

TABLE 4B

MetLeuArgProValGluThrProThrArgGluIleLysLysLeuAspGlyLeu
TrpAlaPheSerLeuAspArgGluArgValAlaAspLeuAsnAlaSerValGln
IleAsnCysThrArgProAsnAsnAsnThrArgLysSerIleThrLysGlyPro
GlyArgValIleTyrAlaThrGlyGlnIleIleGlyAspIleArgLysAlaHis
CysAsnLeuSerArgAlaGlnTrpAsnAsnThrLeuLysGlnValValThrLys
LeuArgGluGlnPheAspAsnLysThrIleValPheThrSerSerSerGlyGly
AspProGluIleValLeuHisSerPheAsnCysGlyGlyGluPhePheTyrCys
AsnThrThrGlnLeuPheAsnSerThrTrpAsnSerThrGluGlySerAsnAsn
ThrGlyGlyAsnAspThrIleThrLeuProCysArgIleLysGlnIleValAsn
MetTrpGlnGluValGlyLysAlaMetTyrAlaProProIleSerGlyGlnIle
LysCysIleSerAsnIleThrGlyLeuLeuLeuThrArgAspGlyGlyGluAsp
ThrThrAsnThrThrGluIleArgArgGlnAlaSerArgGluLeuGluPheLeu
LysThrLysGlyProArgAspThrProIlePheIleGly

TABLE 4C

ATGTTACGTCCTGTAGAAACCCCAACCCGTGAAATCAAAAAACTGGACGGCCTG
TGGGCATTCAGTCTGGATCGCGAACGCGTGGCCGATCTGAATGCATCTGTACAA
ATTAATTGTACAAGACCCAACAACAATACAAGAAAAAGTATAACTAAGGGACCA
GGGAGAGTAATTTATGCAACAGGACAAATAATAGGAGATATAAGAAAAGCACAT
TGTAACCTTAGTAGAGCACAATGGAATAACACTTTAAAACAGGTAGTTACAAAA
TTAAGAGAACAATTTGACAATAAAACAATAGTCTTTACGTCATCCTCAGGAGGG
GACCCAGAAATTGTACTTCACAGTTTTAATTGTGGAGGGGAATTTTTCTACTGT
AATACAACACAACTGTTTAATAGTACTTGGAATAGTACTGAAGGGTCAAATAAC
ACTGGAGGAAATGACACAATCACACTCCCATGCAGAATAAAACAAATTGTAAAC
ATGTGGCAGGAAGTAGGAAAAGCAATGTATGCCCCTCCCATCAGTGGACAAATT
AAATGTATATCAAATATTACAGGGCTACTATTAACAAGAGATGGGGGTGAAGAT
ACAACTAATACTACAGAGATCCGTCGACAAGCTTCCCGGGAGCTGGAATTCTTG
AAGACGAAAGGGCCTCGTGATACTCCTATTTTTATAGGT

TABLE 5

GluAsnPheThrAspAsnAlaLysThrIleIleValHisLeuAsnGlu
SerValGlnIleAsnCysThrArgProAsnTyrAsnLysArgLysArgIleHis
IleGlyProGlyArgAlaPheTyrThrThrLysAsnIleIleGlyThrIleArg
GlnAlaHisCysAsnIleSerArgAlaLysTrpAsnAspThrLeuArgGlnIle
ValSerLysLeuLysGluGlnPheLysAsnLysThrIleValPheAsnGlnSer
SerGlyGlyAspProGluIleValMetHisSerPheAsnCysGlyGlyGluPhe
PheTyrCysAsnThrSerProLeuPheAsnSerThrCysLysIleLysGlnIle
IleAsnMetTrpGlnGluValGlyLysAlaMetTyrAlaProProIleGluGly
GlnIleArgCysSerSerAsnIleThrGlyLeuLeuLeuThrArgAspGlyGly
LysAspThrAspThrAsnAspThr

## TABLE 5A

GATCTGAAAATTTCACAGACAATGCTAAAACCATAATAGTACACCTGAATGAA ACTTTTAAAGTGTCTG-
TTACGATTTTGGTATTATCATGTGGACTTACTT

TCTGTACAAATTAATTGTACAAGACCCAACTACAATAAAAGAAAAAGGATACAT AGACATGTTTAAT-
TAACATGTTCTGGGTTGATGTTATTTTCTTTTTCCTATGTA

ATAGGACCAGGGAGAGCATTTTATACAACAAAAAATATAATAGGAACTATAAGA TATCCTGGTCCCTCTCG-
TAAAATATGTTGTTTTTTATATTATCCTTGATATTCT

CAAGCACATTGTAACATTAGTAGAGCAAAATGGAATGACACTTTAAGACAGATA GTTCGTGTAACATTG-
TAATCATCTCGTTTTACCTTACTGTGAAATTCTGTCTAT

GTTAGCAAATTAAAAGAACAATTTAAGAATAAAACAATAGTCTTTAATCAATCC CAATCGTTTAATTTTCTT-
GTTAAATTCTTATTTTGTTATCAGAAATTAGTTAGG

TCAGGAGGGGACCCAGAAATTGTAATGCACAGTTTTAATTGTGGAGGGGAATTT AGTCCTCCCCTGGGTC-
TTTAACATTACGTGTCAAAATTAACACCTCCCCTTAAA

TTCTACTGTAATACATCACCACTGTTTAATAGTACTTGCAAAATAAAACAAATT AAGATGACATTATGTAGT-
GGTGACAAATTATCATGAACGTTTTATTTTGTTTAA

ATAAACATGTGGCAGGAAGTAGGAAAAGCAATGTATGCCCCTCCCATTGAAGGA TATTTGTACACCGTCC-
TTCATCCTTTTCGTTACATACGGGGAGGGTAACTTCCT

CAAATTAGATGTTCATCAAATATTACAGGGCTACTATTAACAAGAGATGGTGGT GTTTAATCTACAAGTAG-
TTTATAATGTCCCGATGATAATTGTTCTCTACCACCA

AAGGACACGGACACGAACGACACCGA TTCCTGTGCCTGTGCTTGCTGTGGCTCTAG

## TABLE 5B

MetLeuArgProValGluThrProThrArgGluIleLysLysLeuAspGlyLeu
TrpAlaPheSerLeuAspArgGluArgValAlaAspIleIleIleAspGlySerGlu
AsnPheThrAspAsnAlaLysThrIleIleValHisLeuAsnGluSerValGln
IleAsnCysThrArgProAsnTyrAsnLysArgLysArgIleHisIleGlyPro
GlyArgAlaPheTyrThrThrLysAsnIleIleIleGlyThrIleArgGlnAlaHis
CysAsnIleSerArgAlaLysTrpAsnAspThrLeuArgGlnIleValSerLys
LeuLysGluGlnPheLysAsnLysThrIleValPheAsnGlnSerSerGlyGly
AspProGluIleValMetHisSerPheAsnCysGlyGlyGluPhePheTyrCys
AsnThrSerProLeuPheAsnSerThrCysLysIleLysGlnIleIleAsnMet
TrpGlnGluValGlyLysAlaMetTyrAlaProProIleGluGlyGlnIleArg
CysSerSerAsnIleThrGlyLeuLeuLeuThrArgAspGlyGlyLysAspThr
AspThrAsnAspThrGluIleArgArsGlnAlaSerArgGluLeuGluPheLeu
LysThrLysGlyProArgAspThrProIlePheIleGly

## TABLE 5C

ATGTTACGTCCTGTAGAAACCCCAACCCGTGAAATCAAAAAACTGGACGGCCTG
TGGGCATTCAGTCTGGATCGCGAACGCGTGGCCGATATCATCGATGGATCTGAA
AATTTCACAGACAATGCTAAAACCATAATAGTACACCTGAATGAATCTGTACAA
ATTAATTGTACAAGACCCAACTACAATAAAAGAAAAAGGATACATATAGGACCA
GGGAGAGCATTTTATACAACAAAAAATATAATAGGAACTATAAGACAAGCACAT
TGTAACATTAGTAGAGCAAAATGGAATGACACTTTAAGACAGATAGTTAGCAAA
TTAAAAGAACAATTTAAGAATAAAACAATAGTCTTTAATCAATCCTCAGGAGGG
GACCCAGAAATTGTAATGCACAGTTTTAATTGTGGAGGGGAATTTTTCTACTGT
AATACATCACCACTGTTTAATAGTACTTGCAAAATAAAACAAATTATAAACATG
TGGCAGGAAGTAGGAAAAGCAATGTATGCCCCTCCCATTGAAGGACAAATTAGA
TGTTCATCAAATATTACAGGGCTACTATTAACAAGAGATGGTGGTAAGGACACG
GACACGAACGACACCGAGATCCGTCGACAAGCTTCCCGGGAGCTGGAATTCTTG
AAGACGAAAGGGCCTCGTGATACTCCTATTTTTATAGGT

23

## TABLE 6

LeuLysGluAlaValGluIleAsnCysThrArgProAsnAsnAsnThrThrArg
SerIleHisIleGlyProGlyArgAlaPheTyrAlaThrGlyAspIleIleGly
AspIleArgGlnAlaHisCysAsnIleSerArgAlaLysTrpAsnAsnThrLeu
LysGlnIleValIleLysLeuArgAspGlnPheGluAsnLysThrIleIlePhe
AsnArgSerSerGlyGlyAspProGluIleValMetHisSerPheAsnCysGly
GlyGluPhePheTyrCysAsnSerThrGlnLeuPheSerSerThrTrpAsnGly
ThrGluGlySerAsnAsnThrGlyGlyAsnAspThrIleThrLeuProCysArg
IleLysGluIleIleAsnMetTrpGlnGluValGlyLysAlaMetTyrAlaPro
ProIleLysGlyGlnValLysCysSerSerAsnIleThrGlyLeuLeuLeuThr
ArgAspGlyGlyAsnSerLysAsnGlySerLysAsnThr

## TABLE 6A

CTGAAAGAAGCTGTAGAAATTAATTGTACAAGGCCCAACAACAATACAACAAGA      GACTTTCTTC-
GACATCTTTAATTAACATGTTCCGGGTTGTTGTTATGTTGTTCT
AGTATACATATAGGACCAGGGAGAGCATTTTATGCAACAGGAGACATAATAGGA   TCATATGTATATCCTG-
GTCCCTCTCGTAAAATACGTTGTCCTCTGTATTATCCT
GATATAAGACAAGCACATTGTAACATTAGTAGAGCAAAATGGAATAACACTTTA   CTATATTCTGTTCGTG-
TAACATTGTAATCATCTCGTTTTACCTTATTGTGAAAT
AAACAGATAGTTATAAAATTAAGAGACCAATTTGAGAATAAAACAATAATCTTT    TTTGTCTATCAATATTT-
TAATTCTCTGGTTAAACTCTTATTTTGTTATTAGAAA
AATCGATCCTCAGGAGGAGACCCAGAAATTGTAATGCACAGTTTTAATTGTGGA   TTAGCTAGGAGTCCTC-
CTCTGGGTCTTTAACATTACGTGTCAAAATTAACACCT
GGGGAATTTTTCTACTGTAATTCAACACAACTGTTTAGTAGTACTTGGAATGGT      CCCCTTAAAAAGAT-
GACATTAAGTTGTGTTGACAAATCATCATGAACCTTACCA
ACTGAAGGGTCAAATAACACTGGAGGAAATGACACAATCACCCTCCCATGCAGA   TGACTTCCCAGTTTAT-
TGTGACCTCCTTTACTGTGTTAGTGGGAGGGTACGTCT
ATAAAAGAAATTATAAACATGTGGCAGGAAGTAGGAAAAGCAATGTATGCCCCT   TATTTTCTTTAATATTT-
GTACACCGTCCTTCATCCTTTTCGTTACATACGGGGA
CCCATCAAAGGACAAGTTAAATGTTCATCAAATATTACAGGGCTGCTATTAACA      GGGTAGTTTCCTGT-
TCAATTTACAAGTAGTTTATAATGTCCCGACGATAATTGT
AGAGATGGTGGTAATAGCAAGAATGGTAGCAAGAATACAGA   TCTCTACCACCATTATCGTTCTTACCATC-
GTTCTTATGTCTCTAG

## TABLE 6B

MetLeuArgProValGluThrProThrArgGluIleLysLysLeuAspGlyLeu
TrpAlaPheSerLeuAspArgGluArgValAlaAspLeuLysGluAlaValGlu
IleAsnCysThrArgProAsnAsnAsnThrThrArgSerIleHisIleGlyPro
GlyArgAlaPheTyrAlaThrGlyAspIleIleGlyAspIleArgGlnAlaHis
CysAsnIleSerArgAlaLysTrpAsnAsnThrLeuLysGlnIleValIleIleLys
LeuArgAspGlnPheGluAsnLysThrIleIlePheAsnArgSerSerGlyGly
AspProGluIleValMetHisSerPheAsnCysGlyGlyGluPhePheTyrCys
AsnSerThrGlnLeuPheSerSerThrTrpAsnGlyThrGluGlySerAsnAsn
ThrGlyGlyAsnAspThrIleThrLeuProCysArgIleLysGluIleIleAsn
MetTrpGlnGluValGlyLysAlaMetTyrAlaProProIleLysGlyGlnVal
LysCysSerSerAsnIleThrGlyLeuLeuLeuThrArgAspGlyGlyAsnSer
LysAsnGlySerLysAsnThrGluIleArgArgGlnAlaSerArgGluLeuGlu
PheLeuLysThrLysGlyProArgAspThrProIlePheIleGly

## TABLE 6C

ATGTTACGTCCTGTAGAAACCCCAACCCGTGAAATCAAAAAACTGGACGGCCTG
TGGGCATTCAGTCTGGATCGCGAACGCGTGGCCGATCTGAAAGAAGCTGTAGAA
ATTAATTGTACAAGGCCCAACAACAATACAACAAGAAGTATACATATAGGACCA
GGGAGAGCATTTTATGCAACAGGAGACATAATAGGAGATATAAGACAAGCACAT
TGTAACATTAGTAGAGCAAAATGGAATAACACTTTAAAACAGATAGTTATAAAA
TTAAGAGACCAATTTGAGAATAAAACAATAATCTTTAATCGATCCTCAGGAGGA
GACCCAGAAATTGTAATGCACAGTTTTAATTGTGGAGGGGAATTTTTCTACTGT
AATTCAACACAACTGTTTAGTAGTACTTGGAATGGTACTGAAGGGTCAAATAAC
ACTGGAGGAAATGACACAATCACCCTCCCATGCAGAATAAAAGAAATTATAAAC
ATGTGGCAGGAAGTAGGAAAAGCAATGTATGCCCCTCCCATCAAAGGACAAGTT
AAATGTTCATCAAATATTACAGGGCTGCTATTAACAAGAGATGGTGGTAATAGC
AAGAATGGTAGCAAGAATACAGAGATCCGTCGACAAGCTTCCCGGGAGCTGGAA
TTCTTGAAGACGAAAGGGCCTCGTGATACTCCTATTTTTATAGGT

## TABLE 7

LeuAsnGluSerValGluIleAsnCysThrArgProTyrAsnAsnValArgArg
SerLeuSerIleGlyProGlyArgAlaPheArgThrArgGluIleIleGlyIle
IleArgGlnAlaHisCysAsnIleSerArgAlaLysTrpAsnAsnThrLeuLys
GlnIleValGluLysLeuArgGluGlnPheLysAsnLysThrIleValPheAsn
HisSerSerGlyGlyAspProGluIleValThrHisSerPheAsnCysGlyGly
GluPhePheTyrCysAsnSerThrGlnLeuPheAsnSerThrTrpAsnGlyThr
AspIleLysGlyAspAsnLysAsnSerThrLeuIleThrLeuProCysArgIle
LysGlnIleIleAsnMetTrpGlnGlyValGlyLysAlaMetTyrAlaProPro
IleGlnGlyGlnIleArgCysSerSerAsnIleThrGlyLeuLeuLeuThrArg
AspGlyGlyAsnSerSerSerArgGlu

## TABLE 7A

CTGAATGAATCTGTAGAAATTAATTGTACAAGACCCTACAACAATGTAAGAAGA          GACTTACT-
TAGACATCTTTAATTAACATGTTCTGGGATGTTGTTACATTCTTCT
AGTCTATCTATAGGACCAGGGAGAGCATTTCGTACAAGAGAAATAATAGGAATT   TCAGATAGATATCCTG-
GTCCCTCTCGTAAAGCATGTTCTCTTTATTATCCTTAA
ATAAGACAAGCACATTGTAACATTAGTAGAGCAAAATGGAATAACACTTTAAAA          TATTCTGTTCGTG-
TAACATTGTAATCATCTCGTTTTACCTTATTGTGAAATTTT
CAGATAGTTGAGAAATTAAGAGAACAATTTAAGAATAAAACAATAGTCTTTAAT   GTCTATCAACTCTTTAAT-
TCTCTTGTTAAATTCTTATTTTGTTATCAGAAATTA
CATTCCTCAGGAGGGGACCCAGAAATTGTAACGCACAGTTTTAATTGTGGAGGG   GTAAGGAGTCCTCCC-
CTGGGTCTTTAACATTGCGTGTCAAAATTAACACCTCCC
GAATTTTTCTACTGTAATTCAACACAACTGTTTAATAGTACTTGGAATGGTACT          CTTAAAAAGATGACAT-
TAAGTTGTGTTGACAAATTATCATGAACCTTACCATGA
GACATTAAAGGAGATAATAAAAATAGCACACTCATCACACTCCCATGCAGAATA   CTGTAATTTCCTCTAT-
TATTTTTATCGTGTGAGTAGTGTGAGGGTACGTCTTAT
AAACAAATTATAAACATGTGGCAGGGAGTAGGCAAAGCAATGTATGCCCCTCCC   TTTGTTTAATATTTG-
TACACCGTCCCTCATCCGTTTCGTTACATACGGGGAGGG
ATCCAAGGACAAATTAGATGTTCATCAAATATTACAGGGCTGCTATTAACAAGA   TAGGTTCCTGTTTAATC-
TACAAGTAGTTTATAATGTCCCGACGATAATTGTTCT
GATGGTGGTAATAGCAGCAGCAGGGAAGA CTACCACCATTATCGTCGTCGTCCCTTCTCTAG

## TABLE 7B

MetLeuArgProValGluThrProThrArgGluIleLysLysLeuAspGlyLeu
TrpAlaPheSerLeuAspArgGluArgValAlaAspLeuAsnGluSerValGlu
IleAsnCysThrArgProTyrAsnAsnValArgArgSerLeuSerIleGlyPro
GlyArgAlaPheArgThrArgGluIleIleGlyIleIleArgGlnAlaHisCys
AsnIleSerArgAlaLysTrpAsnAsnThrLeuLysGlnIleValGluLysLeu
ArgGluGlnPheLysAsnLysThrIleValPheAsnHisSerSerGlyGlyAsp
ProGluIleValThrHisSerPheAsnCysGlyGlyGluPhePheTyrCysAsn
SerThrGlnLeuPheAsnSerThrTrpAsnGlyThrAspIleLysGlyAspAsn
LysAsnSerThrLeuIleThrLeuProCysArgIleLysGlnIleIleAsnMet
TrpGlnGlyValGlyLysAlaMetTyrAlaProProIleGlnGlyGlnIleArg
CysSerSerAsnIleThrGlyLeuLeuLeuThrArgAspGlyGlyAsnSerSer
SerArgGluGluIleArgArgGlnAlaSerArgGluLeuGluPheLeuLysThr
LysGlyProArgAspThrProIlePheIleGly

## TABLE 7C

ATGTTACGTCCTGTAGAAACCCCAACCCGTGAAATCAAAAAACTGGACGGCCTG
TGGGCATTCAGTCTGGATCGCGAACGCGTGGCCGATCTGAATGAATCTGTAGAA
ATTAATTGTACAAGACCCTACAACAATGTAAGAAGAAGTCTATCTATAGGACCA
GGGAGAGCATTTCGTACAAGAGAAATAATAGGAATTATAAGACAAGCACATTGT
AACATTAGTAGAGCAAAATGGAATAACACTTTAAAACAGATAGTTGAGAAATTA
AGAGAACAATTTAAGAATAAAACAATAGTCTTTAATCATTCCTCAGGAGGGGAC
CCAGAAATTGTAACGCACAGTTTTAATTGTGGAGGGGAATTTTTCTACTGTAAT
TCAACACAACTGTTTAATAGTACTTGGAATGGTACTGACATTAAAGGAGATAAT
AAAAATAGCACACTCATCACACTCCCATGCAGAATAAAACAAATTATAAACATG
TGGCAGGGAGTAGGCAAAGCAATGTATGCCCCTCCCATCCAAGGACAAATTAGA
TGTTCATCAAATATTACAGGGCTGCTATTAACAAGAGATGGTGGTAATAGCAGC
AGCAGGGAAGAGATCCGTCGACAAGCTTCCCGGGAGCTGGAATTCTTGAAGACG
AAAGGGCCTCGTGATACTCCTATTTTTATAGGT

## TABLE 8

LeuAsnGlnSerValGluIleAsnCysThrArgProAsnAsnAsnThrArgLys
SerIleArgIleGlnArgGlyProGlyArgAlaPheValThrIleGlyLysIle
GlyAsn

CTGAACCAATCTGTAGAAATTAATTGTACAAGACCCAACAACAATACAAGAAAA
AGTATCCGTATCCAGAGAGGACCAGGGAGAGCATTTGTTACAATAGGAAAAATA
GGAAAT

## TABLE 8A

MetLeuArgProValGluThrProThrArgGluIleLysLysLeuAspGlyLeu
TrpAlaPheSerLeuAspArgGluArgValAlaAspLeuAsnGlnSerValGlu
IleAsnCysThrArgProAsnAsnAsnThrArgLysSerIleArgIleGlnArg
GlyProGlyArgAlaPheValThrIleGlyLysIleGlyAsn

26

ATGTTACGTCCTGTAGAAACCCCAACCCGTGAAATCAAAAAACTGGACGGCCTG
TGGGCATTCAGTCTGGATCGCGAACGCGTGGCCGATCTGAACCAATCTGTAGAA
ATTAATTGTACAAGACCCAACAACAATACAAGAAAAAGTATCCGTATCCAGAGA
GGACCAGGGAGAGCATTTGTTACAATAGGAAAAATAGGAAAT

TABLE 9 - PEPTIDE 131

Tyr Cys Thr Arg Pro Asn Asn Asn Thr Arg Lys Ser Ile Arg
Ile Gln Arg Cly

TABLE 10 - PEPTIDE 132

Pro Gly Arg Ala Phe Val Thr Ile Gly Lys Ile Gly Asn Met
Arg Gln Ala His Cys Tyr

TABLE 11 - PEPTIDE 134

Glu Arg Val Ala Asp Leu Asn Gln Ser Val Glu Ile Asn Cys
Thr Arg Pro Asn Asn Asn Thr Arg Lys Ser Ile

TABLE 12 - PEPTIDE 135

Asn Asn Thr Arg Lys Ser Ile Arg Ile Gln Arg Gly Pro Gly
Arg Ala Phe Val Thr Ile Gly Lys Ile Gly Cys

TABLE 13 - PEPTIDE 136

Leu Asn Gln Ser Val Glu Ile Asn Cys Thr Arg
Pro Asn Asn Asn Thr Arg Lys Ser Ile Arg Ile
Gln Arg Gly Pro Gly Arg Ala Phe Val Thr Ile
Gly Lys Ile Gly Asn Met

TABLE 14 - PEPTIDE 138

Asn Asn Thr Arg Lys Ser Ile Arg Ile Gln Arg
Gly Pro Gly Arg Val Ile Tyr Ala Thr Gly Lys
Ile Gly Cys

Table 15

5'                         CTGAACCAATCTGTAGAAATTAATTGTACAAGACCCAAC
GACTTGGTTAGACATCTTTAATTAACATGTTCTGGGTTG
AACAATACAAGAAAAAGTATCCGTATCCAGAGAGGACCAGGGAGAGCATTTGTT    TTGTTATGTTCTTTT-
TCATAGGCATAGGTCTCTCCTGGTCCCTCTCGTAAACAA
ACAATAGGAAAAATAGGAAATATGAGACAAGCACATTGTAACATTAGTAGAGCA    TGTTATCCTTTTTATCC-
TTTATACTCTGTTCGTGTAACATTGTAATCATCTCGT
AAATGGAATAACACTTTAAAACAGATAGATAGCAAATTAAGAGAACAATTTGGA    TTTACCTTATTGTGAAAT-
TTTGTCTATCTATCGTTTAATTCTCTTGTTAAACCT
AATAATAAAACAATAATCTTTAAGCAGTCCTCAGGAGGGGACCCAGAAATTGTA    TTATTATTTTGTTAT-
TAGAAATTCGTCAGGAGTCCTCCCCTGGGTCTTTAACAT

27

ACGCACAGTTTTAATTGTGGAGGGGGAATTTTTCTACTGTAATTCAACACAACTG  TGCGTGTCAAAAT-
TAACACCTCCCCTTAAAAAGATGACATTAAGTTGTGTTGAC
TTTAATAGTACTTGGTTTAATAGTACTTGGAGTACTAAAGGGTCAAATAACACT  AAATTATCATGAAC-
CAAATTATCATGAACCTCATGATTTCCCAGTTTATTGTGA
GAAGGAAGTGACACAATCACCCTCCCATGCAGAATAAAACAAATTATAAACATG  CTTCCTTCACTGTGT-
TAGTGGGAGGGTACGTCTTATTTTGTTTAATATTTGTAC
TGGCAGGAAGTAGGAAAAGCAATGTATGCCCCTCCCATCAGTGGACAAATTAGA ACCGTCCTTCATCCTT-
TTCGTTACATACGGGGAGGGTAGTCACCTGTTTAATCT
TGTTCATCAAATATTACAGGGCTGCTATTAACAAGAGATGGTGGTAATAGCAAC ACAAGTAGTTTATAATG-
TCCCGACGATAATTGTTCTCTACCACCATTATCGTTG
AATGAGTCCGA TTACTCAGGCTCTAG      3′

## Claims

1. A protein or peptide selected from the group consisting of
(a) HIV 10 Kd fusion protein denoted Sub 1, having the amino acid sequence shown in Table 2B;
(b) HIV protein portion of Sub 1, having the amino acid sequence shown in Table 2;
(c) HIV 18 Kd fusion protein denoted Sub 2, having the amino acid sequence shown in Table 3B;
(d) HIV protein portion of Sub 2, having the amino acid sequence shown in Table 3;
(e) HIV 27 Kd fusion protein denoted $PB1_{RF}$, having the amino acid sequence shown in Table 4B;
(f) HIV protein portion of $PB1_{RF}$, having the amino acid sequence shown in Table 4;
(g) HIV 28 Kd fusion protein denoted $PB1_{MN}$, having the amino acid sequence shown in Table 5B;
(h) HIV protein portion of $PB1_{MN}$, having the amino acid sequence shown in Table 5;
(i) HIV 26 Kd fusion protein denoted $PB1_{SC}$, having the amino acid sequence shown in Table 6B;
(j) HIV protein portion of $PB1_{SC}$, having the amino acid sequence shown in Table 6;
(k) HIV 26 Kd fusion protein denoted $PB1_{WMJ2}$, having the amino acid sequence shown in Table 7B;
(l) HIV protein portion of $PB1_{WMJ2}$, having the amino acid sequence shown in Table 7;
(m) protein CNBr1, having the amino acid sequence shown in Table 8A;
(n) HIV protein portion of CNBr1, having the amino acid sequence shown in Table 8;
(o) peptide No. 131, having the amino acid sequence shown in Table 9;
(p) peptide No. 132, having the amino acid sequence shown in Table 10;
(q) peptide No. 134, having the amino acid sequence shown in Table 11;
(r) peptide No. 135, having the amino acid sequence shown in Table 12;
(s) peptide No. 136, having the amino acid sequence shown in Table 13;
(t) peptide No. 138, having the amino acid sequence shown in Table 14; and
chemical modifications thereof.

2. A DNA sequence coding for a protein or peptide according to claim 1.

3. A DNA transfer vector comprising DNA according to claim 2.

4. A vector according to claim 3, selected from pPB1-Sub 1, pPB1-Sub2, $pPB1_{RF}$, $pPB1_{MN}$, $pPB1_{SC}$ and $pPB1_{WMJ2}$.

5. A DNA transfer vector of claim 3 or claim 4, transferred to and replicated in a eukaryotic or prokaryotic host.

6. An immunochemical assay for detecting or quantifying antibody against HIV in a fluid, employing a protein or peptide according to claim 1.

7. An immunoadsorbent, for use in solid phase immunochemical assay for antibody against HIV, comprising a solid phase to which is affixed a protein or peptide according to claim 1.

8. An immunoadsorbent of claim 7, further comprising a post-coat of animal protein.

9. A method of detecting antibody against HIV in a biological fluid, comprising the steps of:
(a) incubating a sample of the fluid with an immunoadsorbent according to claim 7, under conditions which allow the anti-HIV antibody in the sample to bind to the immunoadsorbent;
(b) separating the immunoadsorbent from the sample; and
(c) determining if antibody has bound to the immunoadsorbent as an indication of anti-HIV antibody in the sample.

10. A method according to claim 9, wherein step (c) comprises incubating the immunoadsorbent with a labelled antibody against antigen of the species from which the biological fluid is derived; separating the immunoadsorbent from the labelled antibody after the incubation period; and detecting the label associated with the immunoadsorbent.

11. A method according to claim 9, wherein step (c) comprises incubating the immunoadsorbent with at least one labelled protein or peptide according to claim 1; separating the immunoadsorbent from the labelled HIV protein; and detecting the label associated with the immunoadsorbent.

12. A method according to claim 9, wherein step (c) comprises incubating the immunoadsorbent with labelled protein A; separating the immunoadsorbent from the labelled protein A; and detecting the label associated with the immunoadsorbent.

13. A method of detecting antibody against HIV in a human serum or plasma sample, comprising the steps of

(a) incubating the sample with an immunoadsorbent according to claim 7 or claim 8, in the form of a bead coated with the protein or peptide, under conditions which allow anti-HIV antibody in the sample to bind the immunoadsorbent;

(b) separating the immunoadsorbent and the sample;

(c) incubating the immunoadsorbent with a labelled anti-(human IgG) antibody under conditions which allow the anti-(human IgG) antibody to bind human anti-HIV antibody bound to the immunoadsorbent;

(d) separating the immunadsorbent from the unbound anti-(human IgG) antibody; and

(e) evaluating the label associated with the immunoadsorbent as an indication of the presence of antibody against HIV in the sample.

14. A method according to claim 13, wherein the labelled anti-(human IgG) antibody is an animal antibody and the serum or plasma sample is diluted with normal serum of an animal of the same species.

15. A method according to claim 14, wherein the anti-(human IgG) antibody is a goat antibody and the serum or plasma sample is diluted with normal goat serum.

16. A vaccine composition comprising one or more HIV proteins or peptides from one or more HIV isolates.

17. A vaccine composition according to claim 16, comprising a protein or peptide having the antigenic properties of a protein or peptide according to claim 1.

18. A vaccine composition comprising a hybrid protein or peptide having the amino-acid sequence of an HIV protein or peptide from different HIV isolates.

19. A vaccine composition according to claim 18, wherein the HIV isolates are HIV$_{IIIB}$ and HIV$_{RF}$.

20. A kit for use in detecting antibody against HIV in a biological fluid, comprising:

(a) an immunoadsorbent according to claim 7 or claim 8;

(b) labelled HIV antibody; and

(c) means for detecting the label.

21. A kit according to claim 20, wherein the anti-HIV antibody is labelled with anti-(human IgG) antibody as a detectable label.

22. A method for detecting variant-specific viral proteins in biological fluids using antibodies raised to a protein or peptide according to claim 1, which comprises:

(a) incubating an immuno-adsorbed solid phase containing sera capable of binding all HIV envelope proteins with a sample of biological fluid, under conditions which allow antigen in the sample to be bound by the immobilized anti-HIV envelope immunoadsorbent;

(b) separating immunoadsorbent from the sample;

(c) adding HIV variant-specific antibody derived from immunization with the said protein or peptide; and

(d) determining if HIV variant-specific antibody has bound to the immunoadsorbent containing HIV protein.

23. A method according to claim 22, in which detection of HIV variant-specific antibody comprises:

(1) incubating adsorbed HIV envelope proteins with labelled antibody specific for the antigens presented in the HIV variant-specific anti-serum;

(2) separating the immunoadsorbent and labelled antibody after incubation; and

(3) detecting the label associated with the immunoadsorbent.